# EUROPÄISCHE PATENTSCHRIFT

(11) **EP 0 836 593 B1**
(45) Veröffentlichungstag und Bekanntmachung des Hinweises auf die Patenterteilung: **14.02.2001**
(21) Anmeldenummer: 96922037.5
(22) Anmeldetag: 27.06.1996
(51) Int. Cl.: C07D 231/20, A01N 43/56, C07D 403/12

(54) **5-PYRAZOLYLBENZOSÄURE-DERIVATE ALS HERBIZIDE**
5-PYRAZOLYLBENZOIC ACID DERIVATIVES AS HERBICIDES
DERIVES DE 5-ACIDE BENZOIQUE DE PYRAZOLYLE UTILISES COMME HERBICIDES

(30) Priorität: 06.07.1995 DE 19524623
(43) Veröffentlichungstag der Anmeldung: 22.04.1998
(73) Patentinhaber: BASF AKTIENGESELLSCHAFT, 67056 Ludwigshafen (DE)
(72) Erfinder: ZAGAR, Cyrill, D-67061 Ludwigshafen (DE); VON DEM BUSSCHE-HÜNNEFELD, Christoph-Sweder, D-68199 Mannheim (DE); HEISTRACHER, Elisabeth, D-67061 Ludwigshafen (DE); HAMPRECHT, Gerhard, D-69469 Weinheim (DE); KLINTZ, Ralf, D-67269 Gruenstadt (DE); SCHÄFER, Peter, D-67308 Ottersheim (DE); WESTPHALEN, Karl-Otto, D-67346 Speyer (DE); MISSLITZ, Ulf, D-67433 Neustadt (DE); WALTER, Helmut, D-67283 Obrigheim (DE)
(86) Internationale Anmeldenummer: EP9602804
(87) Internationale Veröffentlichungsnummer: WO9702251

(56) Entgegenhaltungen:
- EP-A- 0 443 059
- EP-A- 0 619 946
- DE-A- 4 417 837

## Beschreibung

Die vorliegende Erfindung betrifft neue 5-Pyrazolylbenzoesäure-Derivate der Formel I 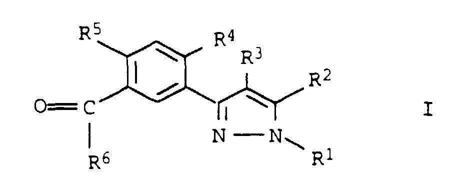 in der die Variablen folgende Bedeutungen haben:
- R¹: Wasserstoff, C₁-C₄-Alkyl, Cyano-C₁-C₄-alkyl oder C₁-C₄-Halogenalkyl;
- R²: C₁-C₄-Alkoxy, C₁-C₄-Alkylthio, C₁-C₄-Halogenalkoxy oder C₁-C₄-Halogenalkylthio;
- R³: Wasserstoff, Cyano, Nitro, Halogen;
- R⁴: Halogen;
- R⁵: Cyano, Halogen, C₁-C₄-Alkyl, C₁-C₄-Halogenalkyl, C₁-C₄-Alkoxy oder C₁-C₄-Halogenalkoxy;
- R⁶: -O-R⁷, -S-R⁷, -N (R⁸)-R⁷ oder -N(R⁸)-O-R⁷;
- R⁷: steht für -X-C(R⁹)=N-O-R¹⁰, -X-C(R⁹) =N-O-Z-R¹⁰, -X-O-N=C(R¹¹,R¹²), -X-SO₂-R¹³ oder für gesättigtes 3- bis 7-gliedriges Heterocyclyl oder Heterocyclyl-C₁-C₄-alkyl, wobei der Heterocyclus neben Methylengliedern ein bis drei Ringglieder enthält, ausgewählt aus der Gruppe bestehend aus drei Azabrücken und zwei Sauerstoff- oder Schwefelatomen, und wobei gewünschtenfalls ein oder zwei Methylengruppen des Heterocyclus durch Carbonyl, Thiocarbonyl und/oder Sulfonyl ersetzt sein können, wobei der Heterocyclus unsubstituiert sein oder ein bis vier Substituenten tragen kann, jeweils ausgewählt aus der Gruppe bestehend aus Halogen, C₁-C₄-Alkyl, C₁-C₄-Alkoxy, (C₁-C₄-Alkoxy)carbonyl, (C₁-C₄-Alkyl)carbonyl, (C₁-C₄-Alkyl)carbonyloxy und (C₁-C₄-Alkyl)carbonylamino;
- R⁸: steht für Wasserstoff, C₁-C₄-Alkyl, C₁-C₄-Halogenalkyl, C₃-C₅-Alkenyl, C₃-C₅-Halogenalkenyl, C₃-C₅-Alkinyl oder C₁-C₄-Alkylsulfonyl;
- R⁹: steht für Wasserstoff, Cyano, C₁-C₄-Alkyl, C₁-C₄-Alkoxy oder C₁-C₄-Alkylthio;
- R¹⁰: steht für Wasserstoff, C₁-C₄-Alkyl, C₁-C₄-Halogenalkyl, C₁-C₄-Cyanoalkyl, Di-(C₁-C₄-alkyl) amino-C₁-C₄-alkyl, C₁-C₄-Alkoxy-C₁-C₄-alkyl, C₁-C₄-Alkylthio-C₁-C₄-alkyl, (C₁-C₄-Alkyl)carbonyl, (C₁-C₄-Alkoxy)carbonyl, Di-(C₁-C₄-alkyl)aminocarbonyl, (C₁-C₄-Halogenalkyl)carbonyl, C₃-C₅-Alkenyl, C₃-C₅-Halogenalkenyl, Di-(C₁-C₄-alkyl)amino-C₃-C₅-alkenyl, C₁-C₄-Alkoxy-C₃-C₅-alkenyl, C₁-C₄-Alkylthio-C₃-C₅-alkenyl, C₃-C₅-Alkinyl, C₃-C₅-Halogenalkinyl, C₃-C₇-Cycloalkyl, das gewünschtenfalls ein bis drei C₁-C₃-Alkylreste tragen kann,
oder für Phenyl, Benzoyl oder 5- oder 6-gliedriges Heteroaryl, das ein bis drei Heteroatome enthält, ausgewählt aus der Gruppe bestehend aus drei Stickstoffatomen und einem Sauerstoff- oder Schwefelatom, wobei die Phenyl- und Heteroarylringe unsubstituiert sein oder an jedem substituierbaren Ringglied einen der folgenden Substituenten tragen können: Nitro, Cyano, Halogen, C₁-C₄-Alkyl, C₁-C₄-Halogenalkyl oder C₁-C₄-Alkoxy;
- R¹¹, R¹²: stehen unabhängig voneinander für Wasserstoff, C₁-C₄-Alkyl, C₁-C₄-Halogenalkyl, C₁-C₄-Alkoxy-C₁-C₄-alkyl, C₃-C₅-Alkenyl, C₃-C₅-Halogenalkenyl, C₃-C₅-Alkinyl, C₃-C₅-Halogenalkinyl, C₃-C₇-Cycloalkyl, das gewünschtenfalls ein bis drei C₁-C₃-Alkylreste tragen kann, oder fur Phenyl, das gewünschtenfalls an jedem substituierbaren Ringglied einen der folgenden Substituenten tragen kann: Nitro, Cyano, Halogen, C₁-C₄-Alkyl, C₁-C₄-Halogenalkyl oder C₁-C₄-Alkoxy,
oder R¹¹ und R¹² bilden zusammen mit dem gemeinsamen Kohlenstoffatom, an das sie gebunden sind, einen gesättigten 3- bis 8-gliedrigen Ring, der neben Methylengliedern gewünschtenfalls ein oder zwei Sauerstoff-, Schwefel- und/oder Azaringglieder enthalten kann, wobei der Ring unsubstituiert sein oder ein bis vier C₁-C₄-Alkylreste tragen kann;
- R¹³: steht für C₁-C₄-Alkyl, C₁-C₄-Halogenalkyl, C₁-C₄-Cyanoalkyl, Di-(C₁-C₄-alkyl)amino-(C₁-C₄-alkyl), C₁-C₄-Alkoxy-C₁-C₄-alkyl, C₁-C₄-Alkylthio-C₁-C₄-alkyl, C₃-C₅-Alkenyl, C₃-C₅-Halogenalkenyl, Di-(C₁-C₄-alkyl)amino-C₃-C₅-alkenyl, C₁-C₄-Alkoxy-C₃-C₅-alkenyl, C₁-C₄-Alkylthio-C₃-C₅-alkenyl, C₃-C₅-Alkinyl, C₃-C₅-Halogenalkinyl, C₃-C₇-Cycloalkyl, das gewünschtenfalls ein bis drei C₁-C₃-Alkylreste tragen kann, für Phenyl oder für 5- oder 6-gliedriges Heteroaryl, das ein bis drei Heteroatome enthält, ausgewählt aus der Gruppe bestehend aus drei Stickstoffatomen und einem Sauerstoff- oder Schwefelatom, wobei der Phenyl- und Heteroarylring unsubstituiert sein oder an jedem substituierbaren Ringglied einen der folgenden Substituenten tragen kann: Nitro, Cyano, Halogen, C₁-C₄-Alkyl, C₁-C₄-Halogenalkyl oder C₁-C₄-Alkoxy;
X und Z stehen unabhängig voneinander für C₁-C₄-Alkylenketten, die unsubstituiert sein oder ein bis vier Substituenten tragen können, jeweils ausgewählt aus der Gruppe bestehend aus Halogen, C₁-C₄-Alkyl, C₁-C₄-Halogenalkyl und C₁-C₄-Alkoxy;
sowie die landwirtschaftlich brauchbaren Salze der Verbindungen I.

Außerdem betrifft die Erfindung die Verwendung dieser Verbindungen als Herbizide, herbizide Mittel, welche die Verbindungen I als wirksame Substanzen enthalten, Verfahren zur Herstellung dieser herbiziden Mittel sowie Verfahren zur Bekämpfung von unerwünschtem Pflanzenwuchs mit den Verbindungen I.

3-Phenylpyrazole vom Typ der Verbindungen I mit einer Ester-, Thioester- oder Säureamidgruppe am Phenylring in meta-Position zum Pyrazol-Rest werden bereits in der WO 92/06 962, EP-A 361 114 und EP-A 443 059 beschrieben.

Des weiteren ist aus der JP-A 03/151 367 bekannt, daß Verbindungen der Formel II 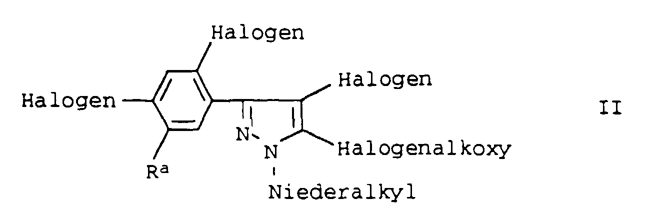 wobei R^{a} u.a. -C(R^{b})₂COR^{c}, -CH=CH-COR^{d} oder -C(COR^{e})=CHR^{f} bedeutet und
- R^{b}: für Wasserstoff oder Niederalkyl,
- R^{c}: für Hydroxy, Niederalkoxy oder Niederalkylthio,
- R^{d}: für Niederalkyl, Niederalkoxy oder Niederalkenyloxy,
- R^{e}: für Niederalkoxy oder Niederalkenyloxy und
- R^{f}: für Hydroxy oder Niederalkoxy stehen,
ebenfalls herbizid wirksam sind.

Außerdem sind gemäß der JP 06/199 805 auch Verbindungen der Formel IIIa 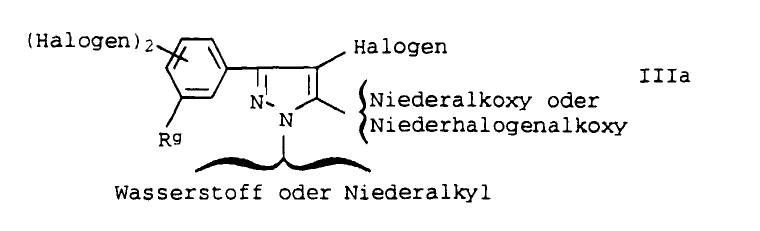 wobei R^{g} u.a. Carboxyl, Niederalkoxycarbonyl oder Niederalkylthiocarbonyl-alkoxycarbonyl bedeutet,
und gemäß der EP-A 447 055 Verbindungen der Formel IIIb 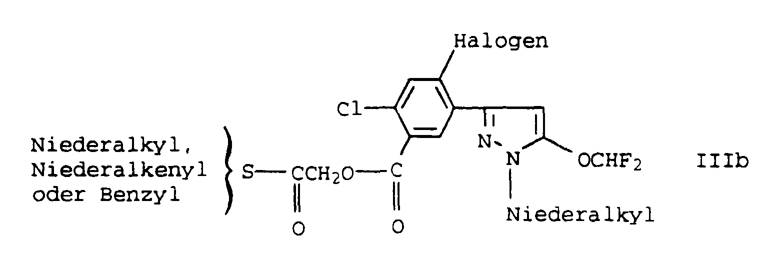 als Herbizide geeignet.

In der EP-A 619 946 werden synergistische herbizide Mittel beschrieben, die 3-Phenylpyrazol-Derivate der Formel IIIc 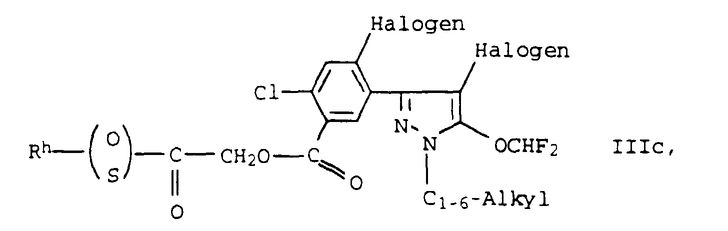 wobei R^{h} für Wasserstoff, C₁₋₆-Alkyl, C₃₋₆-Cycloalkyl, C₁₋₆-Haloalkyl, C₂₋₆-Alkenyl oder C₂₋₆-Alkinyl steht, enthalten.

Gegenstand der EP-A 647 399 ist eine pestizid wirksame, wäßrige Suspension von bestimmten 3-Phenylpyrazolen, u.a. Verbindungen der Formel IIId 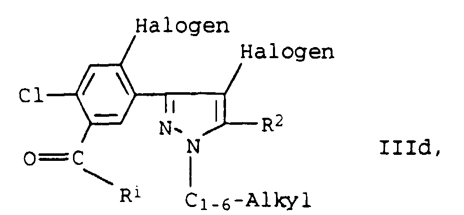 wobei Rⁱ für C₁₋₆-Alkyl, C₁₋₆-Haloalkyl, C₂₋₆-Alkenyl, C₂₋₆-Alkinyl oder -CH(H/C₁₋₆-Alkyl)-CO-(O/S)-R^{k}, mit R^{k} = Wasserstoff, C₁₋₆-Alkyl, C₁₋₆-Haloalkyl, C₂₋₆-Alkenyl oder C₂₋₆-Alkinyl, steht.

Des weiteren werden in der WO 92/02509 bestimmte Phenylpyrazole als herbizide Wirkstoffe beschrieben, die am Phenylring u.a. eine Ester-, Thioester- oder Säureamidgruppe und am Pyrazolring u.a. eine C₁₋₅-Alkylsulfonyl- oder durch Halogen substituierte C₁₋₅-Alkylsulfonylgruppe tragen können. Bei geeigneter Wahl der Substituenten lassen sich Vorprodukte für diese Verbindungen mit Alkylthio oder Halogenalkylthio am Pyrazolring konstruieren, die vom Typ der vorliegenden Verbindungen I sind.

Schließlich werden in der WO 95/30661 Phenyl-thiocarbonsäureamide, die am Phenylring in ortho-Stellung zur Thiocarbonsäureamid-Gruppe u.a. eine Ester-, Thioester- oder Säureamidgruppe und in para-Stellung u.a. einen Pyrazol-3-yl-Ring tragen können, als Herbizide gelehrt. Bei geeigneter Wahl der Substituenten wären die entsprechenden Cyanophenyl-Vorprodukte dieser Verbindungen vom Typ der 5-Pyrazolylbenzoesäure-Derivate I mit R⁵ = Cyano.

Die herbiziden Eigenschaften der bekannten Herbizide bezüglich der Schadpflanzen vermögen jedoch nicht immer voll zu befriedigen. Es lagen daher dieser Erfindung neue, insbesondere herbizid wirksame Verbindungen als Aufgabe zugrunde, mit denen sich unerwünschte Pflanzen besser als bisher gezielt bekämpfen lassen.

Die Aufgabe erstreckt sich auch auf die Bereitstellung neuer desikkant/defoliant wirksamer Verbindungen.

Demgemäß wurden die vorliegenden 5-Pyrazolylbenzoesäure-Derivate der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Defoliation und Desikkation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und Verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Unter landwirtschaftlich brauchbaren Salzen kommen vor allem die Salze derjenigen Kationen oder die Säureadditionssalze derjenigen Säuren in Betracht, deren Kationen beziehungsweise Anionen die herbizide Wirkung der Verbindungen I nicht negativ beeinträchtigen. So kommen als Kationen insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat.

Die für die Substituenten R¹ bis R³, R⁵ und R⁷ bis R¹³ oder als Reste an Alkylenketten, Phenylringen oder Heterocyclen genannten organischen Molekülteile stellen Sammelbegriffe für individuelle Aufzählungen der einzelnen Gruppenmitglieder dar. Sämtliche Kohlenstoffketten, also alle Alkyl-, Cyanoalkyl-, Halogenalkyl-, Alkoxy-, Alkylthio-, Halogenalkoxy-, Halogenalkylthio-, Alkylcarbonyl-, Halogenalkylcarbonyl-, Alkoxycarbonyl-, Alkylcarbonyloxy-, Alkylcarbonylamino-, Alkenyl-, Halogenalkenyl-, Alkinyl-, Halogenalkinyl- und Alkylsulfonyl-Teile können geradkettig oder verzweigt sein. Sofern nicht anders angegeben tragen halogenierte Substituenten vorzugsweise ein bis fünf gleiche oder verschiedene Halogenatome.
Die Bedeutung Halogen steht jeweils für Fluor, Chlor, Brom oder Iod, insbesondere für Fluor oder Chlor.

Ferner bedeuten beispielsweise
- C₁-C₃-Alkyl: Methyl, Ethyl, n-Propyl oder 1-Methylethyl, insbesondere Methyl;
- C₁-C₄-Alkyl sowie die Alkyl-Teile von (C₁-C₄-Alkyl)carbonyl, (C₁-C₄-Alkyl)carbonyloxy, (C₁-C₄-Alkyl)carbonylamino, C₁-C₄-Alkoxy-C₁-C₄-alkyl, C₁-C₄-Alkylthio-C₁-C₄-alkyl, Di-(C₁-C₄-alkyl)aminocarbonyl, Di-(C₁-C₄-alkyl)amino-C₁-C₄-alkyl und Di-(C₁-C₄-alkyl)amino-C₃-C₅-alkenyl: Methyl, Ethyl, n-Propyl, 1-Methylethyl, n-Butyl, 1-Methylpropyl, 2-Methylpropyl oder 1,1-Dimethylethyl, insbesondere Methyl oder Ethyl;
- C₁-C₄-Alkylen: Methylen, Ethan-1,2-diyl, Propan-1,2-diyl, Propan-1,3-diyl, Butan-1,2-diyl, Butan-1,3-diyl, Butan-1,4-diyl oder Butan-2,3-diyl;
- C₁-C₄-Halogenalkyl sowie der Halogenalkyl-Teil von (C₁-C₄-Halogenalkyl)carbonyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl,3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl, insbesondere Trifluormethyl oder 1,2-Dichlorethyl;
- Cyano-C₁-C₄-alkyl: Cyanomethyl, 1-Cyanoethyl, 2-Cyanoethyl, 1-Cyanoprop-1-yl, 2-Cyanoprop-1-yl, 3-Cyanoprop-1-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 3-Cyanobut-2-yl, 3-Cyanobut-2-yl, 4-Cyanobut-2-yl, 1-(Cyanomethyl)-eth-1-yl, 1-(Cyanomethyl)-1-(methyl)-eth-1-yl oder 1-(Cyanomethyl)-prop-1-yl, vorzugsweise Cyanomethyl oder 2-Cyanoethyl;
- Heterocyclyl-C₁-C₄-alkyl: Heterocyclylmethyl, 1-(Heterocyclyl)ethyl, 2-(Heterocyclyl)ethyl, 1-(Heterocyclyl)prop-1-yl, 2-(Heterocyclyl)prop-1-yl, 3-(Heterocyclyl)prop-1-yl, 1-(Heterocyclyl)but-1-yl, 2-(Heterocyclyl)but-1-yl, 3-(Heterocyclyl)but-1-yl, 4-(Heterocyclyl)but-1-y1, 1-(Heterocyclyl)but-2-yl, 2-(Heterocyclyl)but-2-yl, 3-(Heterocyclyl)but-2-yl, 3-(Heterocyclyl)but-2-yl, 4-(Heterocyclyl)but-2-yl, 1-(Heterocyclylmethyl)-eth-1-yl, l-(Heterocyclylmethyl)-1-(methyl)-eth-1-yl oder 1-(Heterocyclylmethyl)-prop-1-yl,vorzugsweise (Heterocyclyl)methyl oder 2-(Heterocyclyl)ethyl;
- C₁-C₄-Alkylsulfonyl: Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl, 1,1-Dimethylethylsulfonyl, n-Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, n-Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl, insbesondere Methylsulfonyl oder Ethylsulfonyl;
- C₁-C₄-Alkoxy sowie die Alkoxy-Teile von (C₁-C₄-Alkoxy)carbonyl, C₁-C₄-Alkoxy-C₁-C₄-alkyl und C₁-C₄-Alkoxy--C₃-C₅-alkenyl: Methoxy, Ethoxy, n-Propoxy, 1-Methylethoxy, n-Butoxy, 1-Methylpropoxy, 2-Methylpropoxy oder 1,1-Dimethylethoxy, insbesondere Methoxy, Ethoxy, 1-Methylethoxy oder 1,1-Dimethylethoxy;
- C₁-C₄-Alkylthio sowie die Alkylthio-Teile von C₁-C₄-Alkylthio-C₁-C₄-alkyl und C₁-C₄-Alkylthio-C₃-C₅-alkenyl: Methylthio, Ethylthio, n-Propylthio, 1-Methylethylthio, n-Butylthio, 1-Methylpropylthio, 2-Methylpropylthio oder 1,1-Dimethylethylthio, insbesondere Methylthio oder Ethylthio;
- C₁-C₄-Halogenalkoxy: C₁-C₄-Alkoxy wie vorstehend genannt, das partiell oder vollständig durch Fluor, Chlor und/oder Brom substituiert ist, also z.B, Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, Bromdifluormethoxy, 1-Fluorethoxy, 2-Fluorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy, vorzugsweise Difluormethoxy, Trifluormethoxy oder Pentafluorethoxy;
- C₁-C₄-Halogenalkylthio: C₁-C₄-Alkylthio wie vorstehend genannt, das partiell oder vollständig durch Fluor, Chlor und/oder Brom substituiert ist, also z.B. Difluormethylthio, Trifluormethylthio, Chlordifluor-methylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio oder 4-Brombutylthio;
- C₃-C₆-Alkenyl sowie die Alkenyl-Teile von C₁-C₄-Alkoxy-C₃-C₆-alkenyl, C₁-C₄-Alkylthio-C₃-C₆-alkenyl und Di-(C₁-C₄-alkyl)amino-C₃-C₆-alkenyl: Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methylprop-1-en-1-yl, 1-Methyl-prop-2-en-1-yl oder 2-Methylprop-2-en-1-yl, n-Penten-1-yl, n-Penten-2-yl, n-Penten-3-yl, n-Penten-4-yl, 1-Methyl-but-1-en-1-yl, 2-Methylbut-1-en-1-yl, 3-Methyl-but-1-en-1-yl, 1-Methylbut-2-en-1-yl, 2-Methyl-but-2-en-1-yl, 3-Methylbut-2-en-1-yl, l-Methyl-but-3-en-1-yl, 2-Methylbut-3-en-1-yl, 3-Methyl-but-3-en-1-yl, 1,1-Dimethylprop-2-en-1-yl, 1,2-Dimethyl-prop-1-en-1-yl, 1,2-Dimethylprop-2-en-1-yl, 1-Ethyl-prop-1-en-2-yl, 1-Ethylprop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, l-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, l,2-Dimetyl-but-2-en-1-yl, 1,2-Dimethyl-but-3-en-1-yl, 1,3-Dimetyl-but-1-en-1-yl, 1,3-Dimethyl-but-2-en-1-yl, 1,3-Dimetyl-but-3-en-1-yl, 2,2-Dimethyl-but-3-en-1-yl, 2,3-Dimetyl-but-1-en-1-yl, 2,3-Dimethyl-but-2-en-1-yl, 2,3-Dimetyl-but-3-en-1-yl, 3,3-Dimethyl-but-1-en-1-yl, 3, 3-Dimetyl-but-2-en-1-yl, 1-Ethyl-but-1-en-1-yl, l-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methylprop-1-en-1-yl oder l-Ethyl-2-methylprop-2-en-1-yl;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, 5 n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methylpent-1-in-5-yl, 4-Methyl-pent-1-in-1-yl, 4-Methylpent-2-in-4-yl oder 4-Methyl-pent-2-in-5-yl;
- C₃-C₆-Halogenalkenyl: C₃-C₆-Alkenyl wie vorstehend genannt, das partiell oder vollständig durch Fluor, Chlor und/oder Brom substituiert ist, also z.B. 2-Chlorallyl, 3-Chlorallyl, 3,3-Dichlorallyl oder Pentafluorallyl, insbesondere C₃- oder C₄-Halogenalkenyl;
- C₃-C₆-Halogenalkinyl: C₃-C₆-Alkinyl wie vorstehend genannt, das partiell oder vollständig durch Fluor, Chlor und/oder Brom substituiert ist, also z.B. 4-Chlorbut-2-in-1-yl;
- C₃-C₇-Cycloalkyl: Cyclopropyl, Cyclobutyl, Cyclopentyl, Cyclohexyl oder Cycloheptyl, insbesondere C₃-C₆-Cycloalkyl;
- gesättigtes 3- bis 7-gliedriges Heterocyclyl sowie der Heterocyclyl-Teil von Heterocyclyl-C₁-C₄-alkyl: z.B. Oxiranyl, Thiiranyl, Aziridin-1-yl, Aziridin-2-yl, Diaziridin-1-yl, Diaziridin-3-yl, Oxetan-2-yl, Oxetan-3-yl, Thietan-2-yl, Thietan-3-yl, Azetidin-1-yl, Azetidin-2-yl, Azetidin-3-yl, Tetrahydrofuran-2-yl, Tetrahydrofuran-3-yl, Tetrahydrothiophen-2-yl, Tetrahydrothiophen-3-yl, Pyrrolidin-1-yl, Pyrrolidin-2-yl, Pyrrolidin-3-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Oxazolidin-2-yl, 1,3-Oxazolidin-3-yl, 1,3-Oxazolidin-4-yl, 1,3-Oxazolidin-5-yl, 1,2-Oxazolidin-2-yl, 1,2-Oxazolidin-3-yl, 1,2-Oxazolidin-4-yl, 1,2-Oxazolidin-5-yl, 1,3-Dithiolan-2-yl, 1,3-Dithiolan-4-yl, Pyrrolidin-1-yl, Pyrrolidin-2-yl, Pyrrolidin-5-yl, Tetrahydropyrazol-1-yl, Tetrahydropyrazol-3-yl, Tetrahydropyrazol-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydropyran-4-yl, Piperidin-1-yl, Piperidin-2-yl, Piperidin-3-yl, Piperidin-4-yl, 1,3-Dioxan-2-yl, 1,3-Dioxan-4-yl, 1,3-Dioxan-5-yl, 1,4-Dioxan-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, Morpholin-2-yl, Morpholin-3-yl, Morpholin-4-yl, Hexahydropyridazin-1-yl, Hexahydropyridazin-3-yl, Hexahydropyridazin-4-yl, Hexahydropyrimidin-1-yl, Hexahydropyrimidin-2-yl, Hexahydropyrimidin-4-yl, Hexahydropyrimidin-5-yl, Piperazin-1-yl, Piperazin-2-yl, Piperazin-3-yl, Hexahydro-1,3,5-triazin-1-yl, Hexahydro-1,3,5-triazin-2-yl, Oxepan-2-yl, Oxepan-3-yl, Oxepan-4-yl, Thiepan-2-yl, Thiepan-3-yl, Thiepan-4-yl, 1,3-Dioxepan-2-yl, 1,3-Dioxepan-4-yl, 1,3-Dioxepan-5-yl, 1,3-Dioxepan-6-yl, 1,3-Dithiepan-2-yl, 1,3-Dithiepan-2-yl, 1,3-Dithiepan-2-yl, 1,3-Dithiepan-2-yl, 1,4-Dioxepan-2-yl, 1,4-Dioxepan-7-yl, Hexahydroazepin-1-yl, Hexahydroazepin-2-yl, Hexahydroazepin-3-yl, Hexahydroazepin-4-yl, Hexahydro-1,3-diazepin-1-yl, Hexahydro-1,3-diazepin-2-yl, Hexahydro-1,3-diazepin-4-yl, Hexahydro-1,4-diazepin-1-yl oder Hexahydro-1,4-diazepin-2-yl;
- 5- oder 6-gliedriges Heteroaryl: insbesondere Furyl wie 2-Furyl und 3-Furyl, Thienyl wie 2-Thienyl und 3-Thienyl, Pyrrolyl wie 2-Pyrrolyl und 3-Pyrrolyl, Isoxazolyl wie 3-Isoxazolyl, 4-Isoxazolyl und 5-Isoxazolyl, Isothiazolyl wie 3-Isothiazolyl, 4-Isothiazolyl und 5-Isothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, des weiteren 2-Pyrazinyl, 1,3,5-Triazin-2-yl und 1,2,4-Triazin-3-yl, insbesondere Pyridyl, Pyrimidyl, Furanyl und Thienyl.

Alle Phenyl- und heterocyclischen Ringe sind vorzugsweise unsubstituiert oder tragen einen Halogen-, Methyl-, Trifluormethyl- oder Methoxy-Substituenten.

Im Hinblick auf die Verwendung der 5-Pyrazolylbenzoesäure-Derivate I als Herbizide sind Verbindungen I bevorzugt, bei denen die Substituenten folgende Bedeutung haben, und zwar jeweils für sich allein oder in Kombination:
- R¹: Wasserstoff oder C₁-C₄-Alkyl, insbesondere C₁-C₄-Alkyl; besonders bevorzugt ist Methyl;
- R²: C₁-C₄-Halogenalkoxy, insbesondere Difluormethoxy;
- R³: Wasserstoff oder Halogen, insbesondere Halogen; besonders bevorzugt ist Chlor;
- R⁴: Fluor oder Chlor;
- R⁵: Halogen oder C₁-C₄-Halogenalkyl, insbesondere Halogen; besonders bevorzugt ist Chlor;
- R⁶: -O-R⁷ oder -N(R⁸)-R⁷;
- R⁷: -X-C(R⁹)=N-O-Z-R¹⁰, -X-O-N=C(R¹¹,R¹²), -X-SO₂-^{R13} oder gesättigtes 3- bis 6-gliedriges Heterocyclyl oder Heterocyclyl-C₁-C₄-alkyl, wobei der Heterocyclus neben Methylengliedern ein bis drei Ringglieder enthält, ausgewählt aus der Gruppe bestehend aus drei Azabrücken und zwei Sauerstoff- oder Schwefelatomen, und wobei gewünschtenfalls ein oder zwei Methylengruppen des Heterocyclus durch Carbonyl, Thiocarbonyl und/oder Sulfonyl ersetzt sein können, wobei der Heterocyclus unsubstituiert sein oder ein bis vier Substituenten tragen kann, jeweils ausgewählt aus der Gruppe bestehend aus Halogen, C₁-C₄-Alkyl, C₁-C₄-Alkoxy, (C₁-C₄-Alkoxy)carbonyl, (C₁-C₄-Alkyl)carbonyl, (C₁-C₄-Alkyl)carbonyloxy und (C₁-C₄-Alkyl)carbonylamino; insbesondere -X-C(R⁹)=N-O-Z-R¹⁰ oder -X-O-N=C(R¹¹,R¹²);
- R⁸: Wasserstoff oder C₁-C₄-Alkyl, insbesondere Wasserstoff oder Methyl;
- R⁹: Wasserstoff oder C₁-C₄-Alkyl, insbesondere Wasserstoff oder Methyl;
- R¹⁰: Wasserstoff, C₁-C₄-Alkyl, C₁-C₄-Halogenalkyl, C₁-C₄-Alkoxy-C₁-C₄-alkyl, C₃-C₅-Alkenyl, C₃-C₅-Halogenalkenyl, C₃-C₅-Alkinyl, C₃-C₅-Halogenalkinyl, C₃-C₇-Cycloalkyl, das gewünschtenfalls ein bis drei C₁-C₃-Alkylreste tragen kann, oder Phenyl, Benzoyl oder 5- oder 6-gliedriges Heteroaryl, das ein bis drei Heteroatome enthält, ausgewählt aus der Gruppe bestehend aus drei Stickstoffatomen und einem Sauerstoff- oder Schwefelatom, wobei die Phenyl- und Heteroarylringe unsubstituiert sein oder an jedem substituierbaren Ringglied einen der folgenden Substituenten tragen können: Nitro, Cyano, Halogen, C₁-C₄-Alkyl, C₁-C₄-Halogenalkyl oder C₁-C₄-Alkoxy;
insbesondere Wasserstoff, C₁-C₄-Alkyl, C₁-C₄-Halogenalkyl, C₃-C₅-Alkenyl, C₃-C₅-Alkinyl, Phenyl, Benzoyl oder 5- oder 6-gliedriges Heteroaryl, das ein bis drei Heteroatome enthält, ausgewählt aus der Gruppe bestehend aus drei Stickstoffatomen und einem Sauerstoff- oder Schwefelatom, wobei die Phenyl- und Heteroarylringe unsubstituiert sein oder an jedem substituierbaren Ringglied einen der folgenden Substituenten tragen können: Nitro, Cyano, Halogen oder C₁-C₄-Alkyl;
- R¹¹, R¹²: unabhängig voneinander
Wasserstoff, C₁-C₄-Alkyl, C₁-C₄-Halogenalkyl, C₃-C₅-Alkenyl, C₃-C₅-Alkinyl, C₃-C₇-Cycloalkyl oder Phenyl, das gewünschtenfalls an jedem substituierbaren Ringglied einen der folgenden Substituenten tragen kann: Nitro, Cyano, Halogen und
C₁-C₄-Alkyl,
insbesondere C₁-C₄-Alkyl,
oder R¹¹ und R¹² bilden zusammen mit dem gemeinsamen Kohlenstoffatom, an das sie gebunden sind, einen gesättigten 5- oder 6-gliedrigen carbocyclischen Ring;
- R¹³: C₁-C₄-Alkyl, C₁-C₄-Halogenalkyl, C₁-C₄-Alkoxy-C₁-C₄-alkyl, C₃-C₅-Alkenyl, C₃-C₅-Halogenalkenyl, C₃-C₅-Alkinyl, Phenyl oder 5- oder 6-gliedriges Heteroaryl, das ein bis drei Heteroatome enthält, ausgewählt aus der Gruppe bestehend aus drei Stickstoffatomen und einem Sauerstoff- oder Schwefelatom, wobei der Phenyl- und Heteroarylring unsubstituiert sein oder an jedem substituierbaren Ringglied einen Halogen-oder C₁-C₄-Alkyl-Substituenten tragen kann, insbesondere C₁-C₄-Alkyl oder Phenyl;
- X und Z: unabhängig voneinander C₁-C₄-Alkylenketten, die unsubstituiert sein oder ein bis vier Halogen- und/oder C₁-C₄-Alkyl-Substituenten tragen können, insbesondere Methylen, Ethan-1,2-diyl oder Propan-1,3-diyl.

Ganz besonders bevorzugt sind die in der folgenden Tabelle 1 aufgeführten Verbindungen Ia (≙ I mit R¹ = Methyl; R² = Difluormethoxy; R³, R⁴ und R⁵ = Chlor):

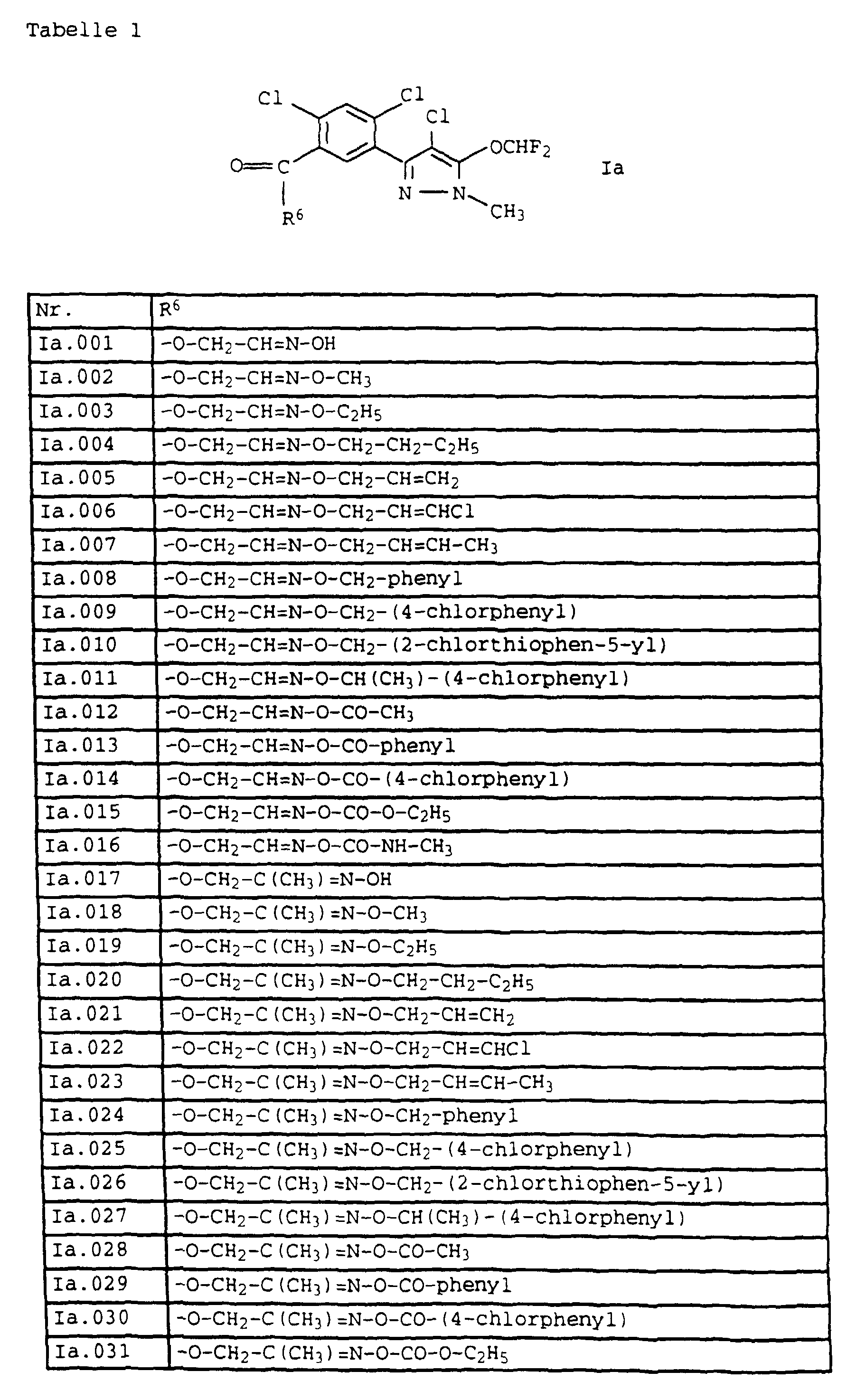

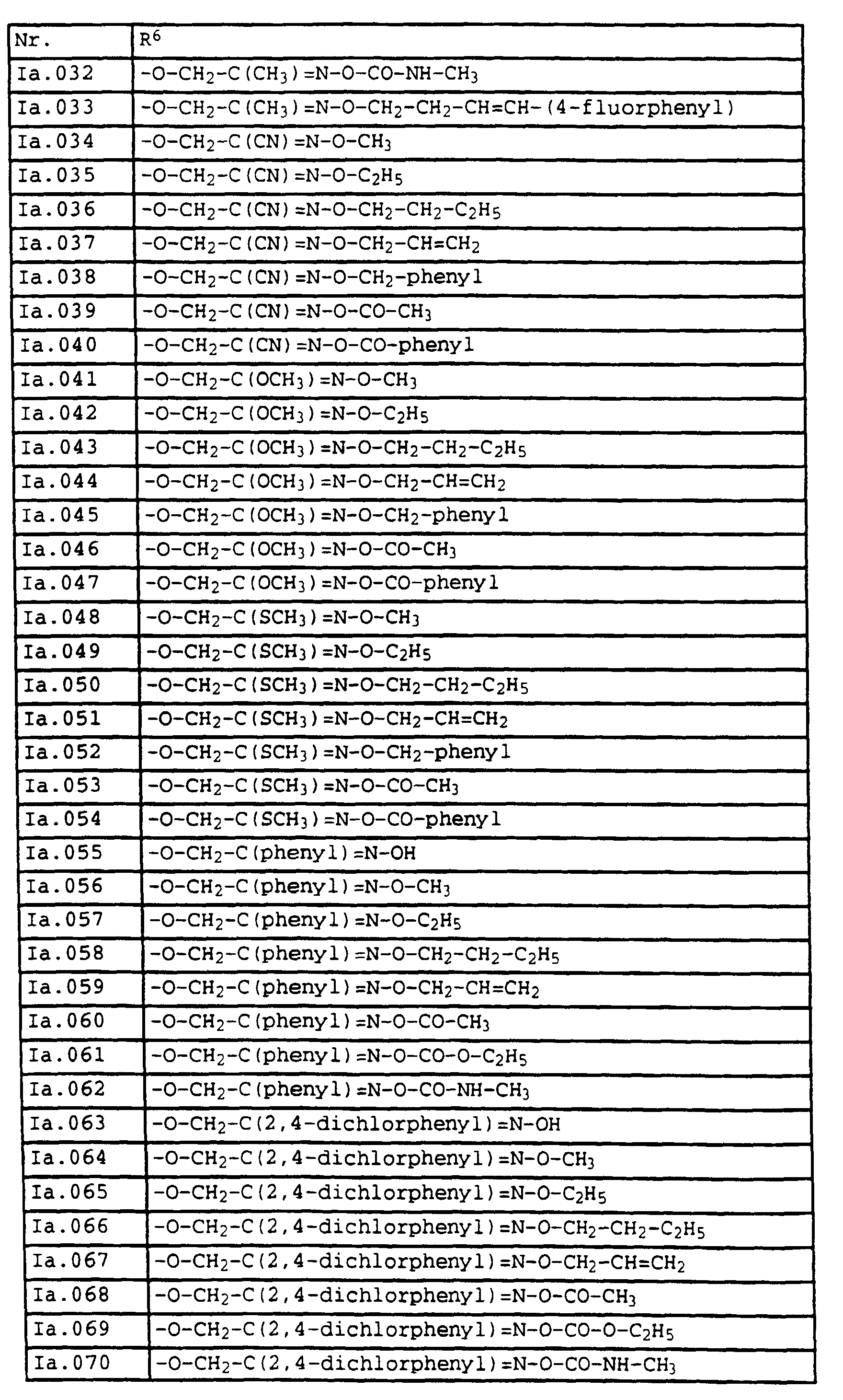

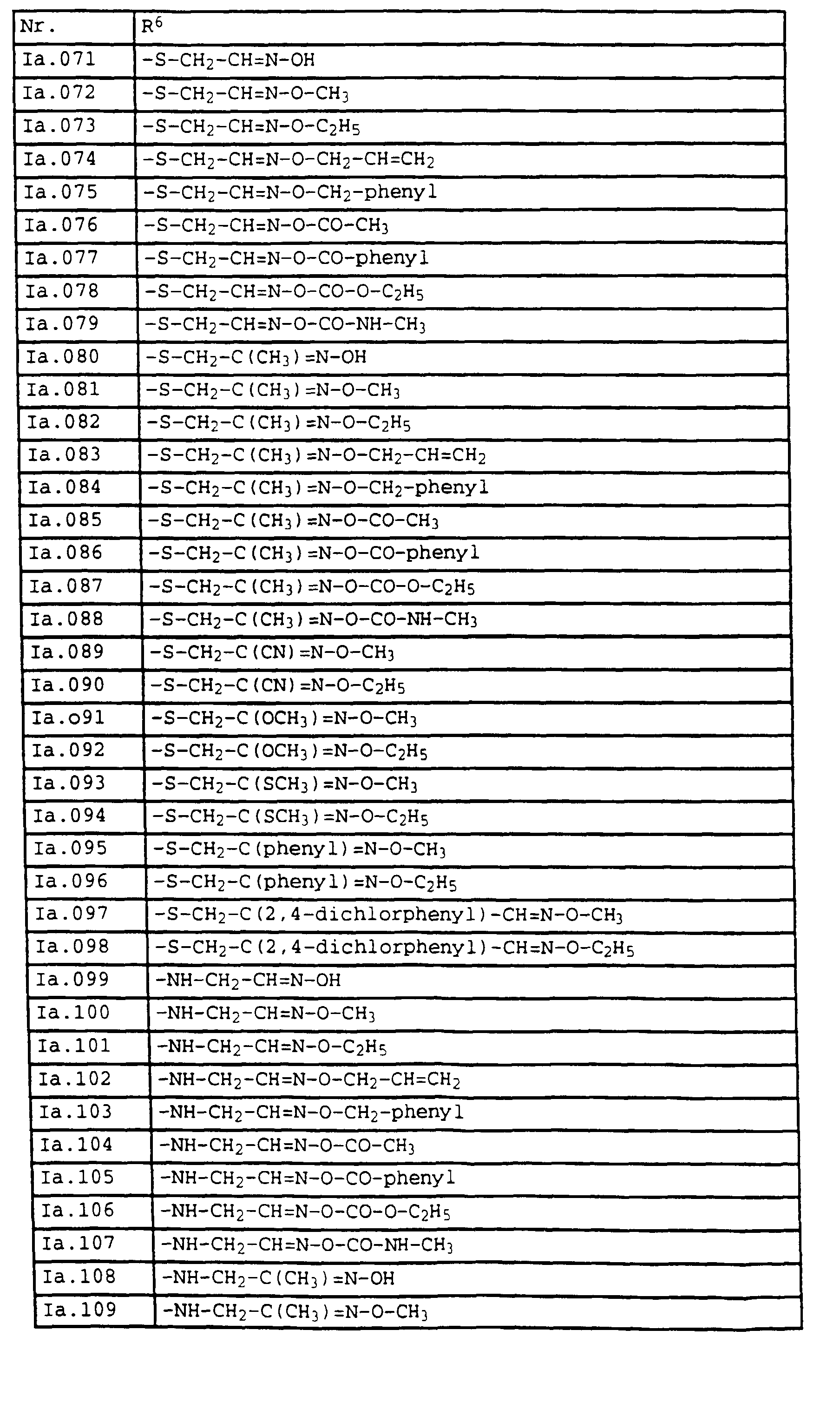

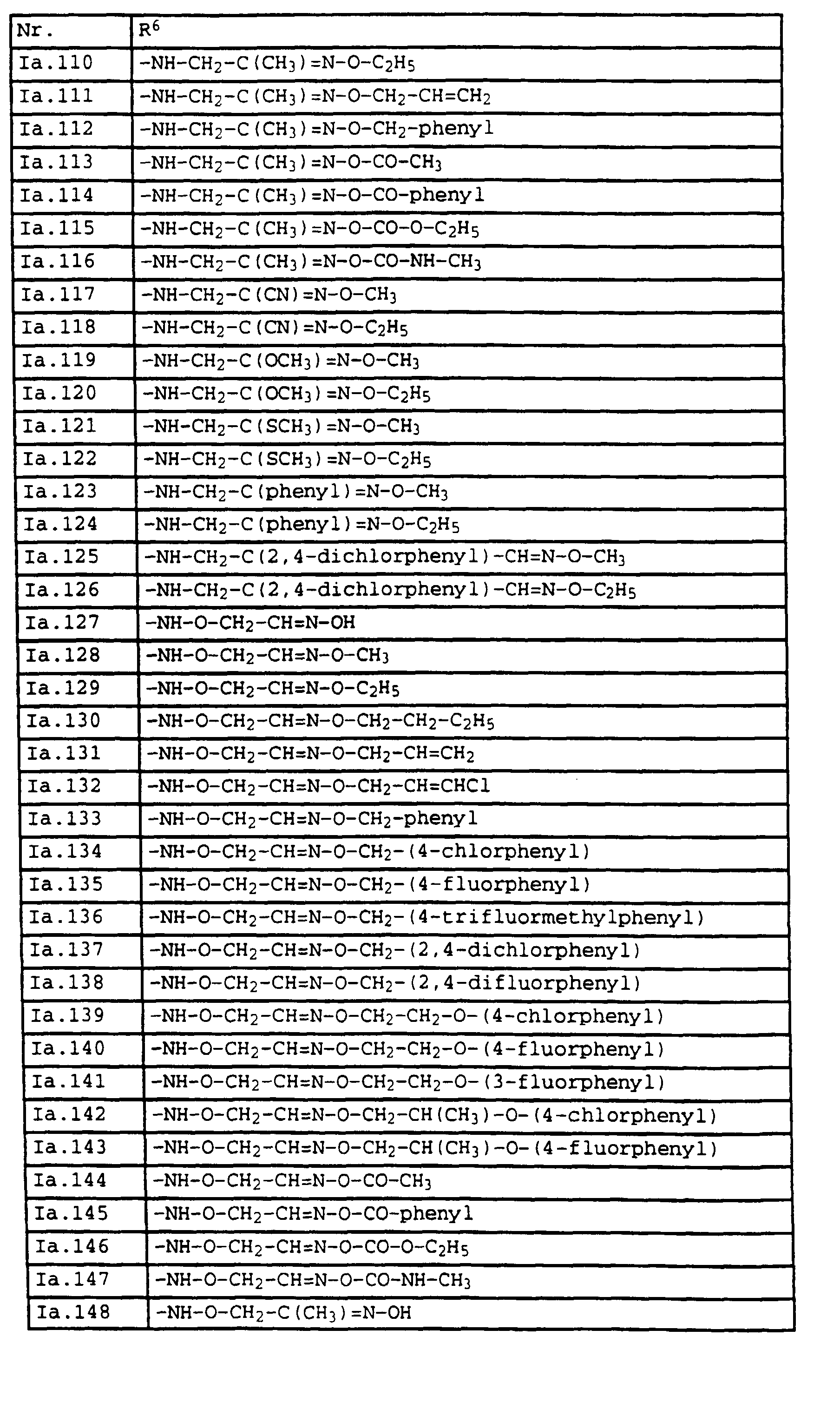

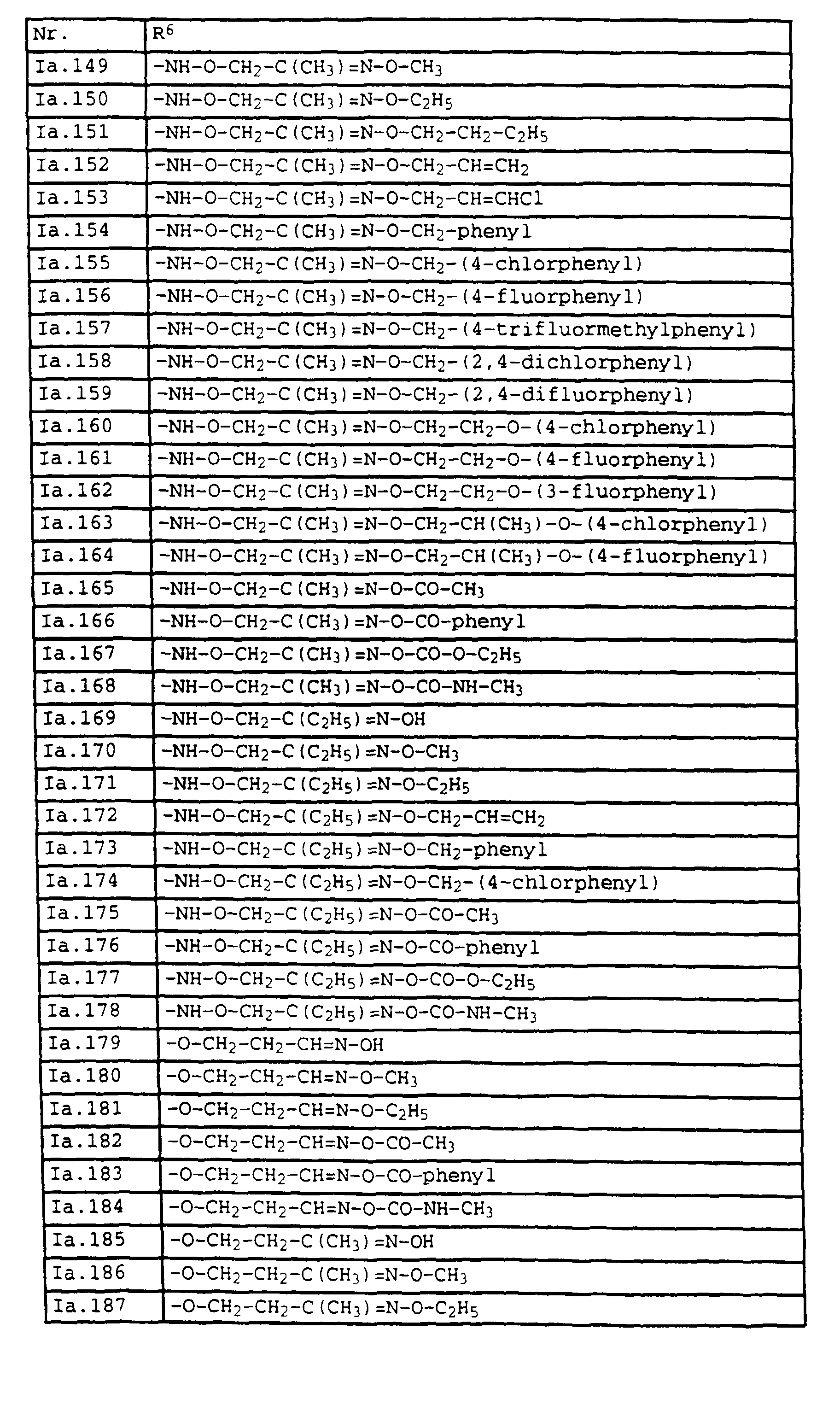

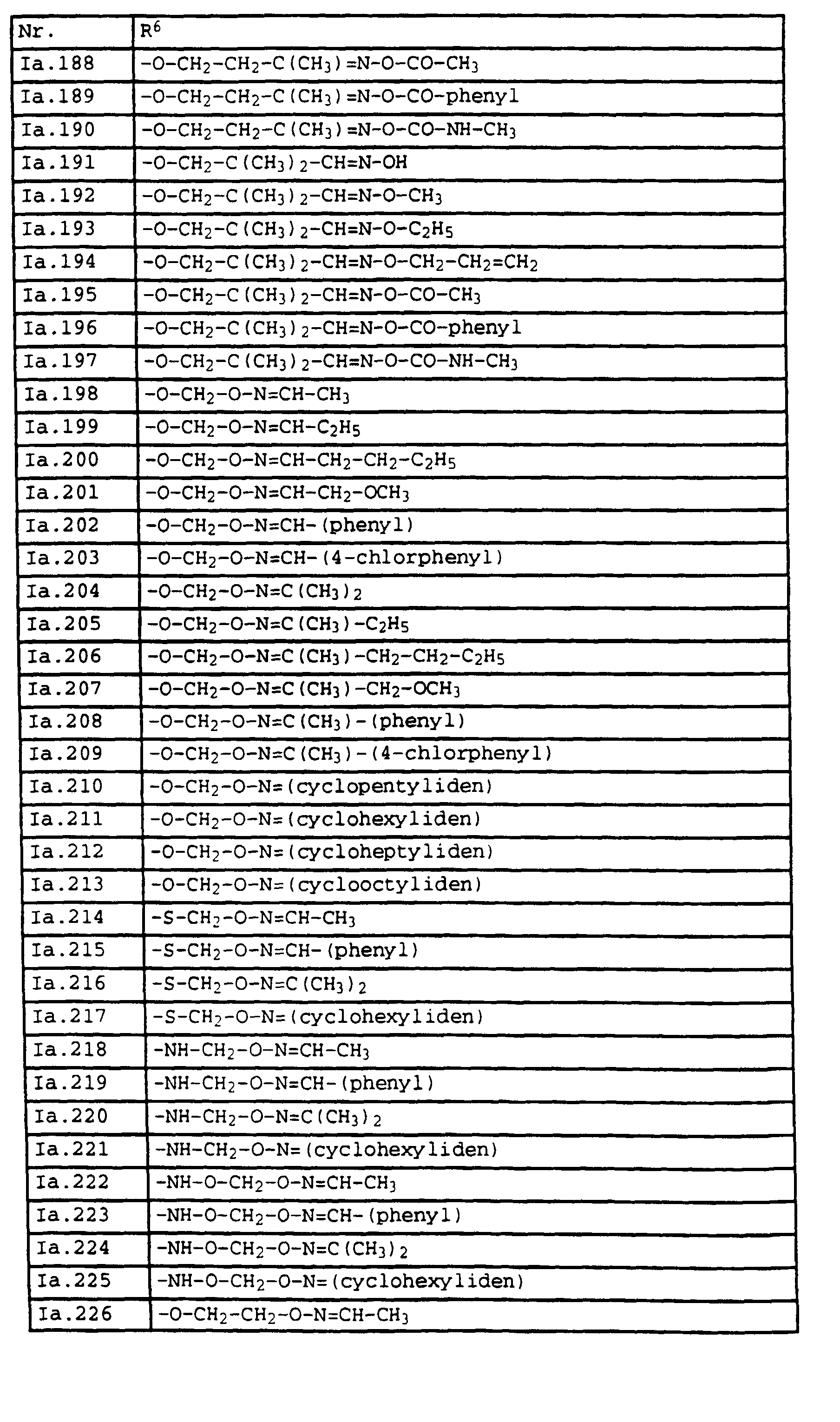

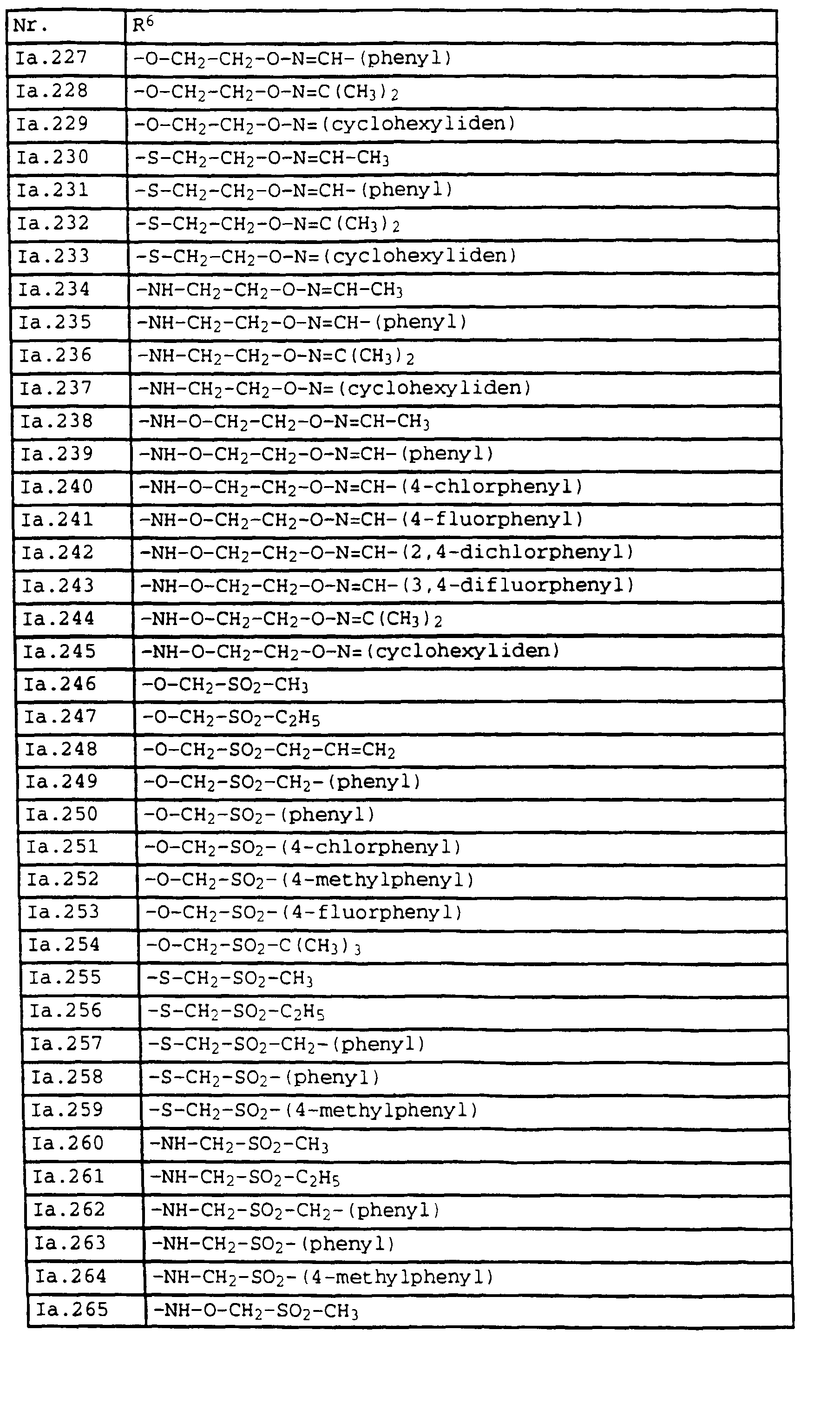

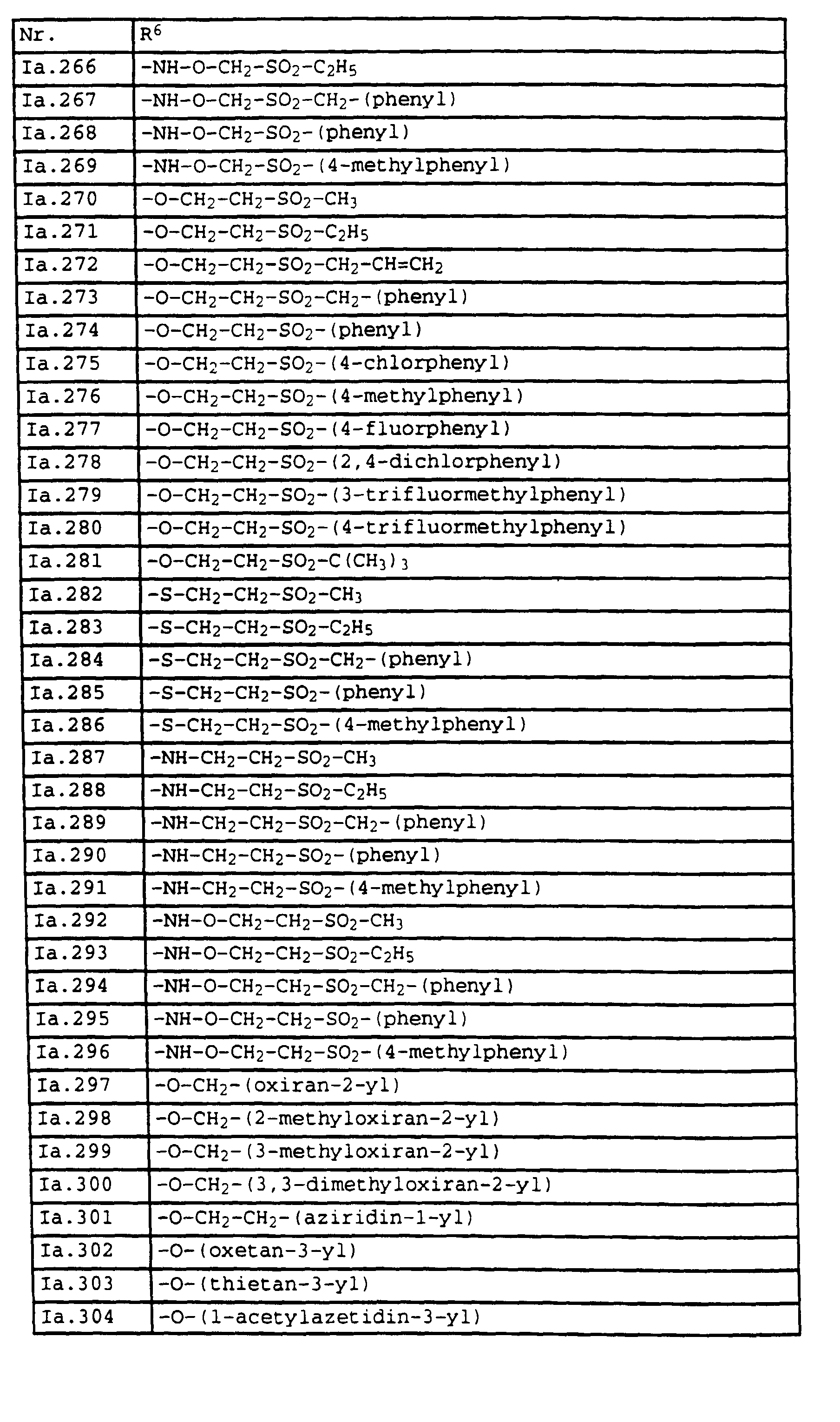

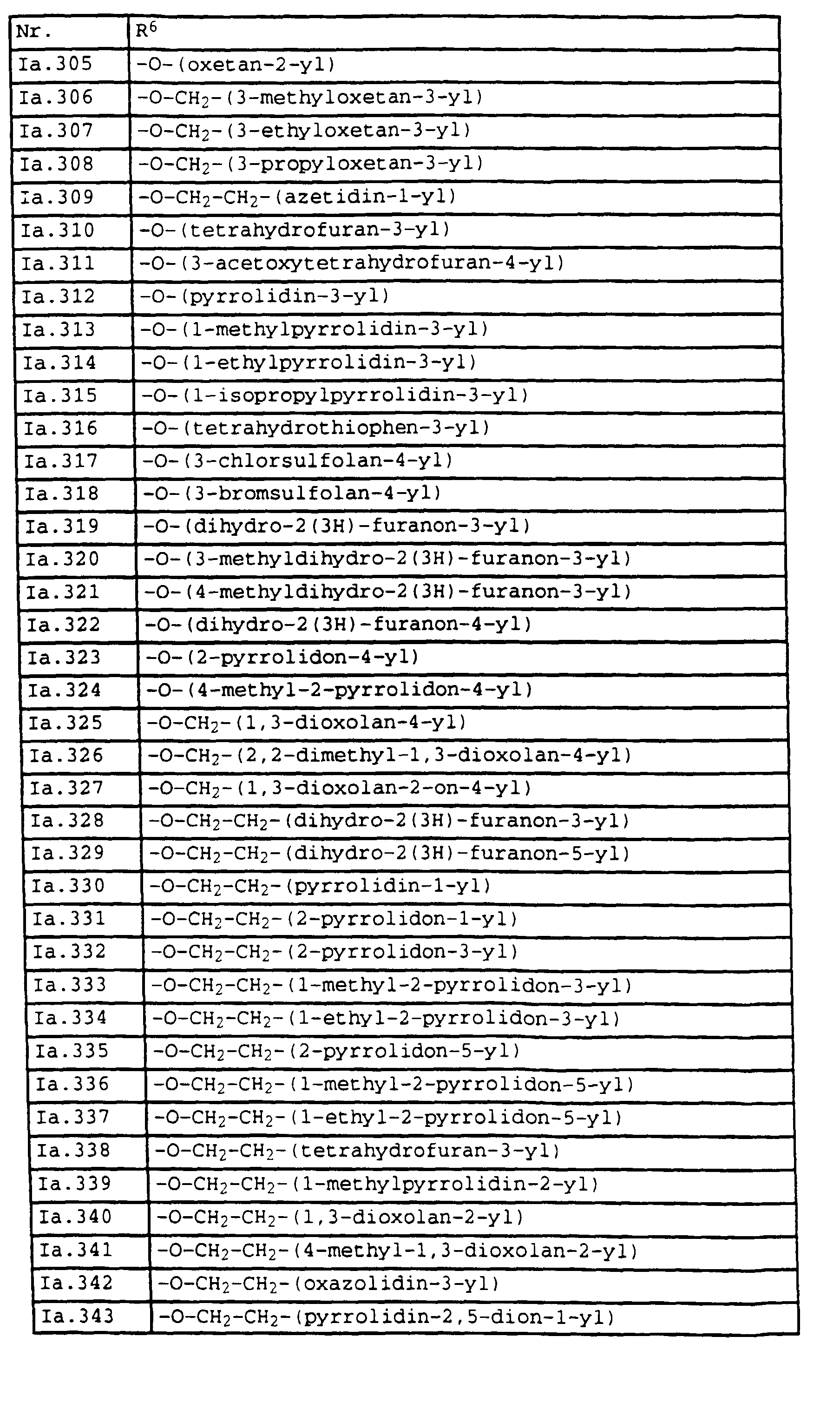

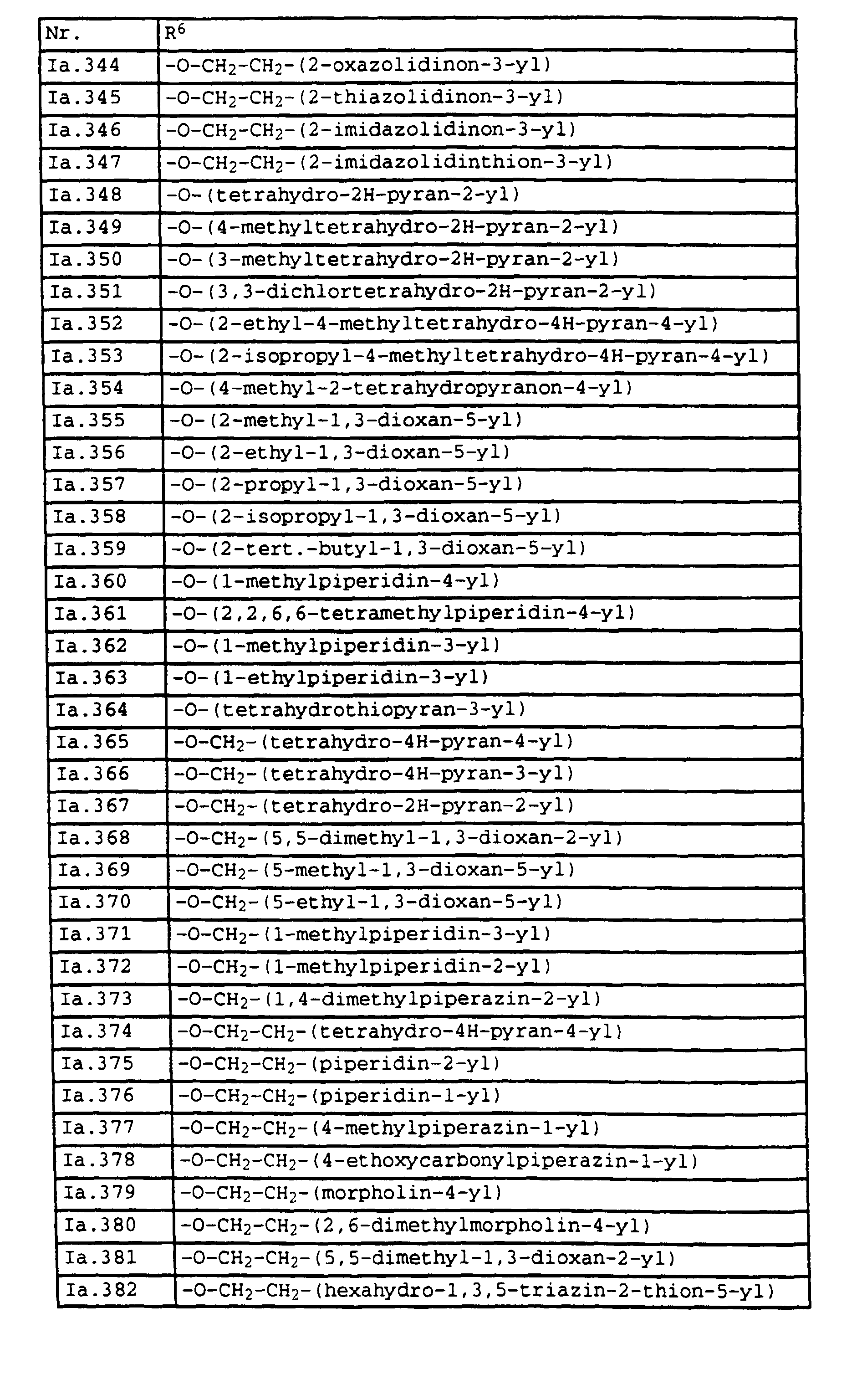

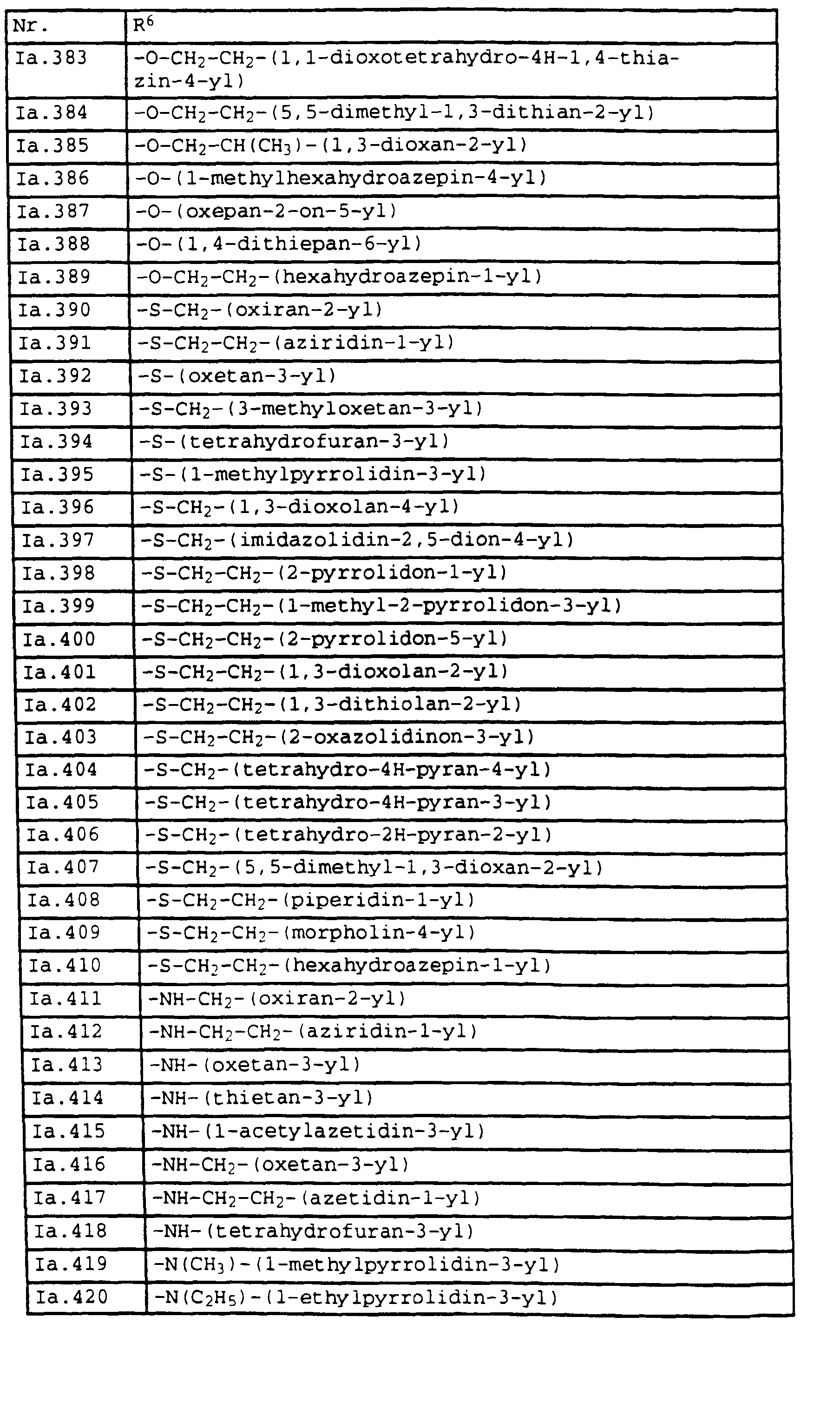

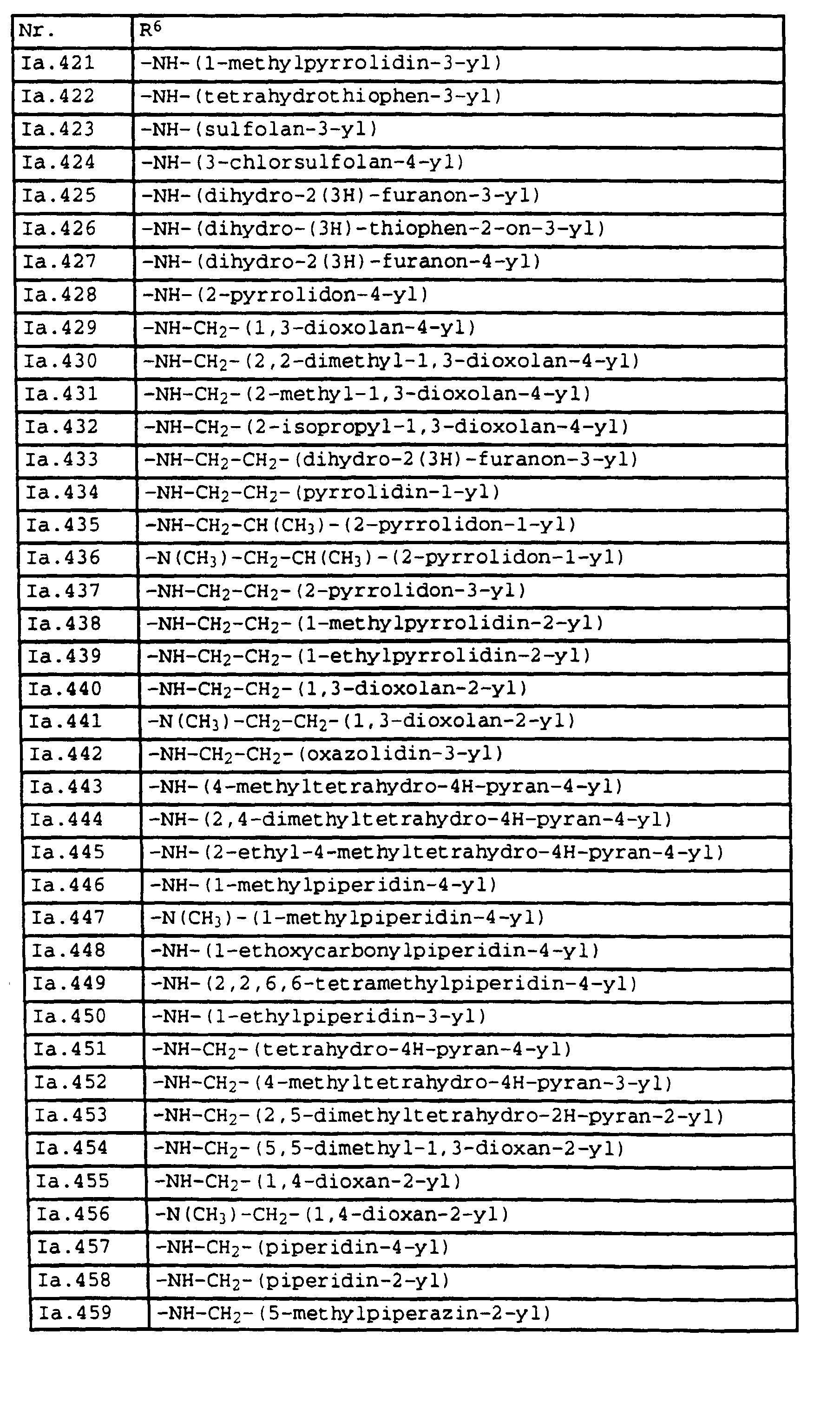

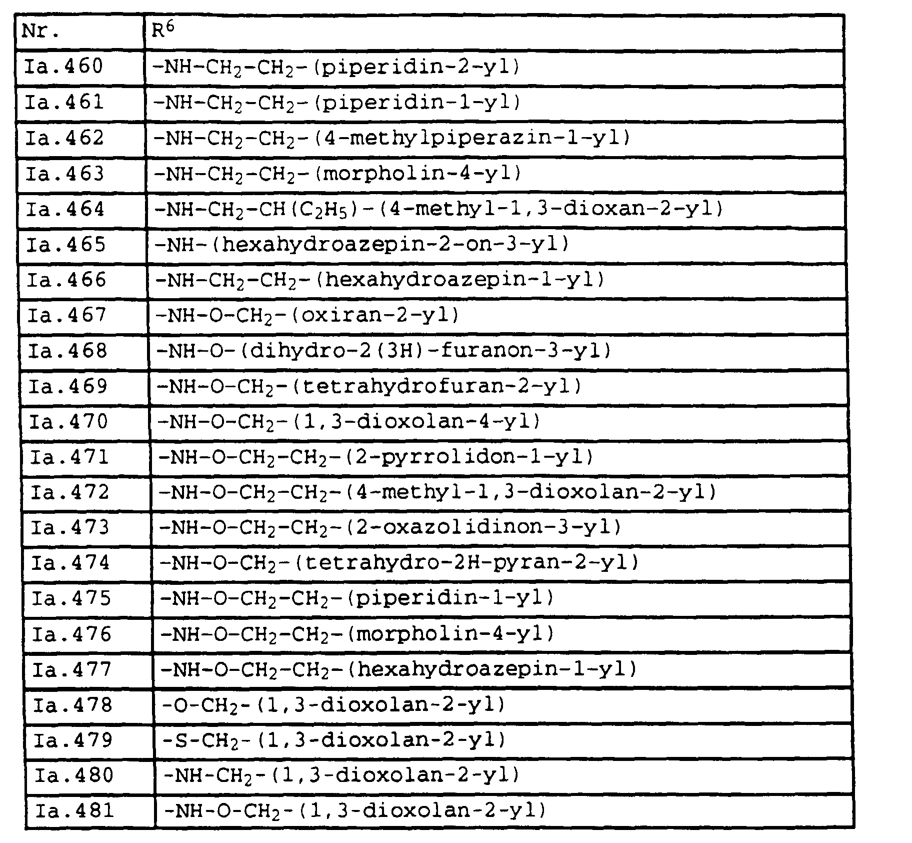

Des weiteren sind die 5-Pyrazolylbenzoesäure-Derivate der Formel Ib besonders bevorzugt, insbesondere die Verbindungen Ib.001 - Ib.481, die sich von den entsprechenden Verbindungen Ia.001 - Ia.481 lediglich dadurch unterscheiden, daß R⁴ für Fluor steht:

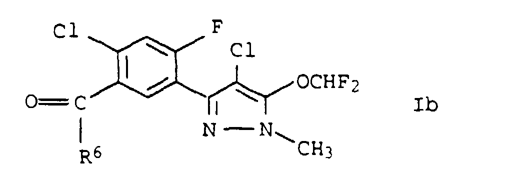

Die 5-Pyrazolylbenzoesäure-Derivate der Formel I sind auf verschiedene Weise erhältlich, insbesondere nach einem der folgenden Verfahren:
A) Umsetzung eines β-Ketocarbonsäurederivats IV mit Hydrazin oder einem Hydrazin-Derivat in einem inerten Lösungsmittel (vgl. z.B. JP-A 04/225 937 und JP-A 03/072 460) und Alkylierung des Verfahrensproduktes V: 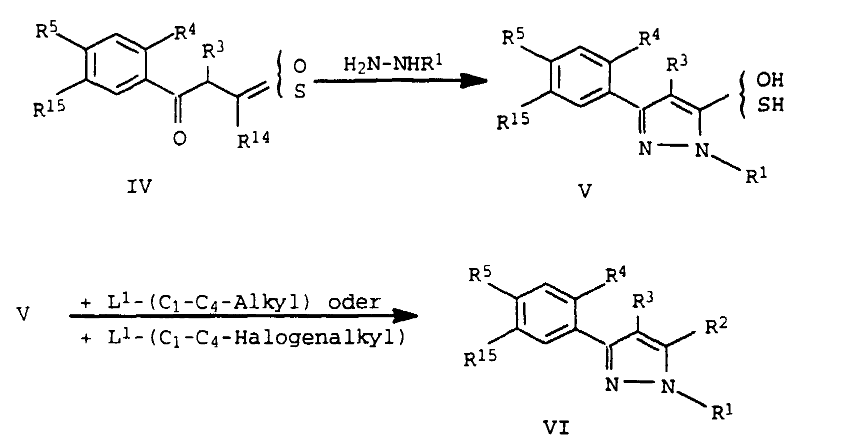
   - L¹: steht für eine übliche Abgangsgruppe wie Halogen, -O-SO₂CH₃, -O-SO₂CF₃, -O-SO₂C₄F₉ und -O-SO₂(p-CH₃-C₆H₄);
   - R¹⁴: steht vorzugsweise für Halogen, C₁-C₄-Alkoxy oder (C₁-C₄-Alkyl)carbonyloxy;
   - R¹⁵: steht für Wasserstoff, Nitro, Amino, Halogen, Alkyl, Halogenalkyl, -CH₂-R⁶, Formyl, Carboxy, Alkoxycarbonyl oder für -CO-R⁶.

   Das Lösungsmittel kann aprotisch oder protisch sein. Geeignet sind z.B. organische Säuren wie Essigsäure, Kohlenwasserstoffe, halogenierte Kohlenwasserstoffe, Ether wie Ethylenglykoldimethylether, Alkohole wie Methanol und Ethanol, sowie Sulfoxide. Es kann jedoch auch ohne Lösungsmittel gearbeitet werden.
   Die Reaktionstemperatur wird hauptsächlich vom Schmelzpunkt des Lösungsmittels oder der Verbindung IV und dem Siedepunkt des Reaktionsgemisches vorgegeben. Bevorzugt arbeitet man bei etwa 60 bis 120°C.
   Im allgemeinen setzt man etwa die 0,95- bis 5-fache molare Menge, vorzugsweise die 1- bis 1,4-fache Menge, an Hydrazin oder Hydrazin-Derivat, bezogen auf das β-Ketocarbonsäurederivat IV, ein.
   Die Menge an Alkylierungsmittel L¹-(C₁-C₄-Alkyl) oder L¹-(C₁-C₄-Halogenalkyl) liegt üblicherweise ebenfalls bei 0,95- bis zur 5-fachen molaren Menge, bezogen auf das Zwischenprodukt V.
   Mit Blick auf die bevorzugten Reste R¹ an den 3-Phenylpyrazolen I sind unter den Hydrazin-Derivaten diejenigen besonders bevorzugt, die eine Alkylgruppe tragen.
   Die Alkylierung erfolgt normalerweise mit dem Halogenid, vorzugsweise dem Chlorid oder Bromid, oder mit dem Sulfat eines Alkans oder Halogenalkans, gewünschtenfalls in Gegenwart einer organischen Base, z.B. eines Trialkylamins oder von Pyridin, oder einer anorganischen Base, z.B. eines Alkalimetallcarbonats.
   Zweckmäßigerweise wir die Alkylierung in einem inerten organischen Lösungsmittel vorgenommen, z.B. in einem aliphatischen oder cyclischen Ether wie 1,2-Dimethoxyethan, Tetrahydrofuran und Dioxan, in einem aliphatischen Keton wie Aceton, in einem Amid wie Dimethylformamid, in einem Sulfoxid wie Dimethylsulfoxid oder in einer Mischung aus einem dieser Lösungsmittel und Wasser.
   Die Reaktion ist im allgemeinen bei einer Temperatur von 0°C bis zur Siedetemperatur des Reaktionsgemisches ausführbar. Vorzugsweise arbeitet man bei etwa 20 bis 80°C.
B) Halogenierung der Verbindungen VI mit R³ = Wasserstoff: 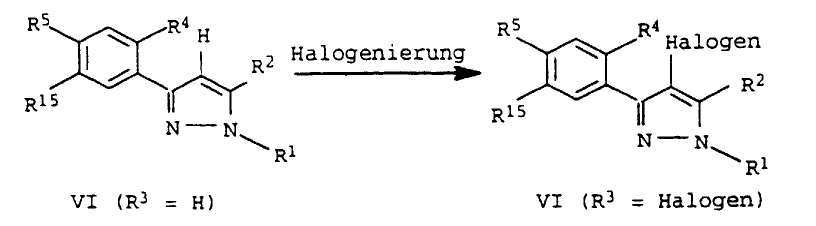
   Die Umsetzung kann in einem inerten Lösungs-/Verdünnungsmittel oder lösungsmittelfrei vorgenommen werden.
   Beispiele für geeignete Lösungsmittel sind organische Säuren, anorganische Säuren, Kohlenwasserstoffe, halogenierte Kohlenwasserstoffe, aromatische Kohlenwasserstoffe, Ether, Sulfide, Sulfoxide und Sulfone.
   Als Halogenierungsmittel kommen beispielsweise Chlor, Brom, N-Bromsuccinimide, N-Chlorsuccinimide oder Sulfurylchlorid in Betracht. Je nach Ausgangsverbindung und Halogenierungsmittel kann der Zusatz eines Radikalstarters, beispielsweise eines organischen Peroxides wie Dibenzoylperoxid oder einer Azoverbindung wie Azobisisobutyronitril, oder Bestrahlung mit Licht vorteilhaft auf den Reaktionsverlauf wirken.
   Die Menge an Halogenierungsmittel ist nicht kritisch. Sowohl unterstöchiometrische Mengen als auch große Überschüsse an Halogenierungsmittel, bezogen auf die zu halogenierende Verbindung VI mit R³ = Wasserstoff, sind möglich.
   Bei Verwendung eines Radikalstarters ist üblicherweise eine katalytische Menge ausreichend.
   Die Reaktionstemperatur liegt normalerweise bei (- 100) bis 200°C, vornehmlich bei 10 bis 100°C oder dem Siedepunkt des Reaktionsgemisches.
C) Halogenierung von 3-(3-Methylphenyl)pyrazolen VI (R¹⁵ = CH₃) und
   a) nucleophile Substitution an den Verfahrensprodukten mit anschließender Oxidation oder
   b) Hydrolyse der Verfahrensprodukte:

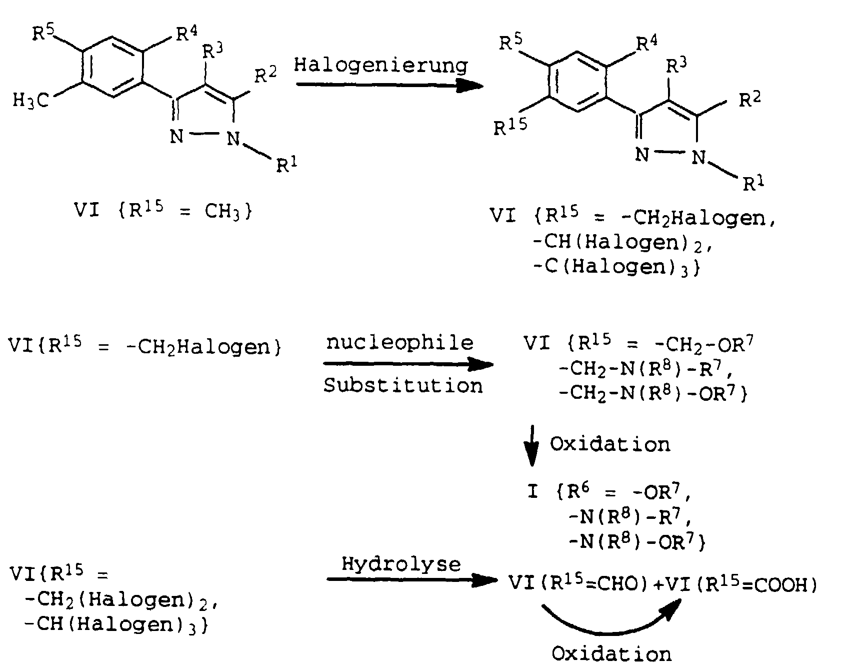

   Bezüglich der Lösunsmittel, Mengenverhältnisse und der Reaktionsbedingungen bei der Halogenierung sei auf die Angaben unter Methode B) verwiesen.
   Bei den halogenierten Verbindungen VI mit R¹⁵ = Halogenmethyl kann das Halogenatom durch einen Alkohol- (-O-R⁷) oder Amin-Rest (-N(R⁸)-R⁷ oder -N(R⁸)-O-R⁷) nucleophil substituiert werden.
   Als Nucleophil werwendet man entweder die entsprechenden Alkohole oder Amine, wobei dann vorzugsweise in Gegenwart einer Base (z.B. eines Alkali- oder Erdalkalimetallhydroxids oder Alkali- oder Erdalkalimetallcarbonats) gearbeitet wird, oder man verwendet die durch Reaktion der Alkohole oder Amine mit einer Base (z.B. einem Alkalimetallhydrid) erhaltenen Alkalimetallsalze dieser Verbindungen.
   Als Lösungsmittel kommen vor allem aprotische organische Solventien, z.B. Tetrahydrofuran, Dimethylformamid, Dimethylsulfoxid, oder Kohlenwasserstoffe wie Toluol und Hexan, in Betracht.
   Die Reaktionsführung erfolgt bei einer Temperatur zwischen dem Schmelz- und dem Siedepunkt des Reaktionsgemisches, vorzugsweise bei 0 bis 100°C.
   Die Substitutionsprodukte können auf an sich bekannte Weise {siehe z.B. Houben-Weyl, Methoden der Organischen Chemie, Bd. E5, Georg Thieme Verlag, Stuttgart 1985, S. 935 f.; S.J. Angyal, K. James, Carbohydr. Res. 12, 147 (1970); P.F. Schuda, M.B. Cichowicz, M.R. Heimann, Tetrahedron Lett. 24, 3829 (1983); ibid. 24, 4267 (1983)} durch Oxidation in die entsprechenden 5-Pyrazolylbenzoesäure-Derivate I (R⁶ = -O-R⁷, -N(R⁸)-R⁷ oder -N(R⁸)-O-R⁷) übergeführt werden.
   Als Oxidationsmittel kommen hierfür vorzugsweise Übergangsmetallverbindungen, z.B. Oxide des Chroms, Rhodiums oder Rutheniums, in Betracht, vorzugsweise in katalytischer bis stöchiometrischer Menge, bezogen auf die zu oxidierende Verbindung VI. Bei der Verwendung katalytischer Mengen werden zusätzliche Oxidationsmittel benötigt, beispielsweise Sauerstoff, Amin-N-oxide oder Alkalimetallperiodate, in stöchiometrischen Mengen, oder, z.B. zur Erzielung besonders hoher Umsätze, auch im Überschuß.
   Neben den üblichen organischen Solventien eignen sich hierfür organische Säuren als Lösungsmittel. Die Oxidation kann auch in einem wäßrig-organischen Zweiphasensystem durchgeführt werden, dann vorzugsweise in Gegenwart eines Phasentransferkatalysators.
   Die Reaktionstemperatur liegt in der Regel zwischen dem Schmelz- und Siedepunkt des Reaktionsgemisches, vorzugsweise bei 0 bis 100°C.
   Die Hydrolyse der Verbindungen VI mit R¹⁵ = Dihalogen- oder Trihalogenmethyl erfolgt vorzugsweise unter sauren Bedingungen, beispielsweise lösungsmittelfrei in Salzsäure, Essigsäure, Ameisensäure oder Schwefelsäure, oder auch in wäßrigen Lösungen der genannten Säuren.
   Die Hydrolyse wird in der Regel bei einer Temperatur zwischen dem Schmelz- und Siedepunkt des Reaktionsgemisches durchgeführt, vorzugsweise bei 0 bis 10°C.
   Die Oxidation der Hydrolyseprodukte VI mit R¹⁵ = Formyl zu den entsprechenden Carbonsäuren kann auf an sich bekannte Weise erfolgen (siehe hierzu insbesondere die Seiten 179 bis 181 von A.H. Haines, "Methods for the Oxidation of Organic Compounds", Academic Press, 1988).
D) "Sandmeyer"-Reaktion von Anilinen VI (R¹⁵ = NH₂) auf an sich bekannte Weise (siehe z.B. Houben-Weyl, Methoden der Organischen Chemie, Bd. 5/4, Georg Thieme Verlag, Stuttgart 1960, S. 438ff.): 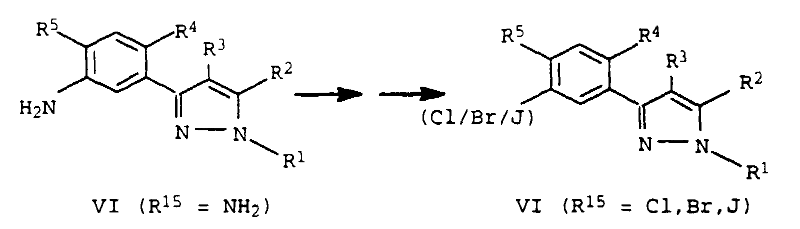
   Im allgemeinen erhält man das Diazoniumsalz auf an sich bekannte Weise durch Umsetzung des Anilins VI (R¹⁵ = NH₂) in einer wäßrigen Säurelösung, z.B. in Salzsäure, Bromwasserstoffsäure oder Schwefelsäure, mit einem Nitrit wie Natriumnitrit und Kaliumnitrit.
   Es besteht aber auch die Möglichkeit, wasserfrei, z.B. in chlorwasserstoffhaltigem Eisessig, in absolutem Alkohol, in Dioxan oder Tetrahydrofuran, in Acetonitril oder in Aceton zu arbeiten und hierbei das Anilin VI (R¹⁵ = NH₂) mit einem Salpetrigsäureester wie tert.-Butylnitrit und Isopentylnitrit zu behandeln.
   Die Überführung des so erhaltenen Diazoniumsalzes in die Halogenverbindung VI mit R¹⁵ = Chlor, Brom oder Jod erfolgt besonders bevorzugt durch Behandeln mit einer Lösung oder Suspension eines Kupfer(I)salzes wie Kupfer(I)chlorid, -bromid und -jodid, oder mit einer Alkalimetallsalz-Lösung.
   Diese Umsetzung des Diazoniumsalzes kann z.B. in Wasser, in wässriger Salzsäure oder Bromwasserstoffsäure, in einem Keton wie Aceton, Diethylketon und Methylethylketon, in einem Nitril wie Acetonitril, in einem Ether wie Dioxan und Tetrahydrofuran oder in einem Alkohol wie Methanol und Ethanol erfolgen.
   Die Reaktionstemperatur liegt normalerweise bei (- 30) bis + 50°C.
   Bevorzugt werden alle Reaktionspartner in etwa stöchiometrischen Mengen eingesetzt, jedoch kann ein Überschuß der einen oder anderen Komponente von Vorteil sein.

Die Aniline VI mit R¹⁵ = Amino sind durch Reduktion der entsprechenden Nitroverbindungen VI mit R¹⁵ = Nitro erhältlich:

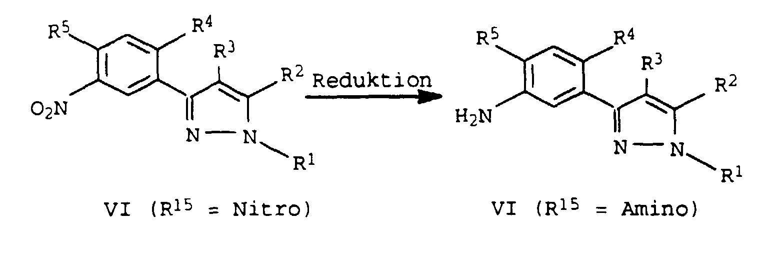

Die Reduktion kann mit einem Metall wie Eisen, Zink oder Zinn unter sauren Reaktionsbedingungen oder mit einem komplexen Hydrid wie Lithiumaluminiumhydrid und Natriumborhydrid erfolgen, wobei als Lösungsmittel - in Abhängigkeit vom gewählten Reduktionsmittel - z.B. Wasser, Alkohole wie Methanol, Ethanol und Isopropanol oder Ether wie Diethylether, Methyltert.-butylether, Dioxan, Tetrahydrofuran und Ethylenglykoldimethylether, in Betracht kommen.

Bei der Reduktion mit einem Metall arbeitet man vorzugsweise lösungsmittelfrei in einer anorganischen Säure, insbesondere in konzentrierter oder verdünnter Salzsäure, oder in einer organischen Säure wie Essigsäure. Es ist aber auch möglich, der Säure ein inertes Lösungsmittel, z.B. eines der vorstehend genannten, zuzumischen.

Die Ausgangsverbindung VI (R¹⁵ = NO₂) und das Reduktionsmittel werden zweckmäßigerweise in etwa äquimolaren Mengen eingesetzt; zur Optimierung des Reaktionsverlaufes kann es jedoch vorteilhaft sein, eine der beiden Komponenten im Überschuß zu verwenden, bis etwa zur 10-fachen molaren Menge.

Die Menge an Säure ist nicht kritisch. Um die Ausgangsverbindung möglichst vollständig zu reduzieren verwendet man zweckmäßigerweise mindestens eine äquivalente Menge an Säure.

Die Reaktionstemperatur liegen im allgemeinen bei (-30) bis 200°C, bevorzugt bei 0 bis 80°C.

Zur Aufarbeitung wird die Reaktionsmischung üblicherweise mit Wasser verdünnt und das Produkt durch Filtration, Kristallisation oder Extraktion mit einem Lösungsmittel, das mit Wasser weitgehend unmischbar ist, z.B. mit Essigsäureethylester, Diethylether oder Methylenchlorid, isoliert. Gewünschtenfalls kann das Produkt anschließend wie üblich gereinigt werden.

Die Nitrogruppe der Nitrophenylpyrazole VI (R¹⁵ = Nitro) kann auch katalytisch mittels Wasserstoff hydriert werden. Hierfür geeignete Katalysatoren sind beispielsweise Raney-Nickel, Palladium auf Kohle, Palladiumoxid, Platin und Platinoxid, wobei im allgemeinen eine Katalysatormenge von 0,05 bis 10,0 mol-%, bezogen auf die zu reduzierende Verbindung, ausreichend ist.

Man arbeitet entweder lösungsmittelfrei oder in einem inerten Lösungs- oder Verdünnungsmittel, z.B. in Essigsäure, einem Gemisch aus Essigsäure und Wasser, Essigsäureethylester, Ethanol oder in Toluol.

Nach Abtrennen des Katalysators kann die Reaktionslösung wie üblich auf das Produkt hin aufgearbeitet werden.

Die Hydrierung kann bei Normaldruck oder unter erhöhtem Druck durchgeführt werden.

Die Nitroverbindungen VI mit R¹⁵ = Nitro ihrerseits sind bevorzugt durch Nitrierung von Phenylpyrazolen VI mit R¹⁵ = H zugänglich:

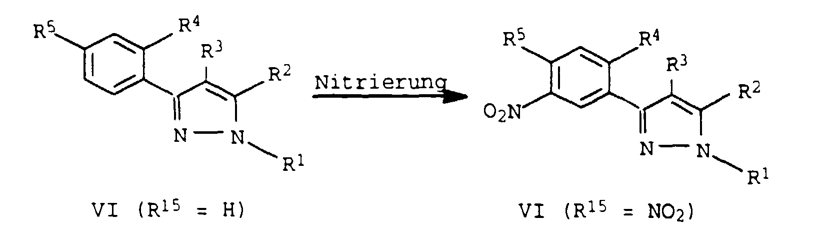

Als Nitrierungs-Reagenzien kommen beispielsweise Salpetersäure in unterschiedlicher Konzentration, auch konzentrierte und rauchende Salpetersäure, Mischungen von Schwefelsäure und Salpetersäure, Acetylnitrate und Alkylnitrate in Betracht.

Die Reaktion kann entweder lösungsmittelfrei in einem Überschuß des Nitrier-Reagenzes oder in einem inerten Lösungs- oder Verdünnungsmittel durchgeführt werden, wobei z.B. Wasser, Mineralsäuren, organische Säuren, Chlorkohlenwasserstoffe wie Methylenchlorid, Anhydride wie Essigsäureanhydrid und Mischungen dieser Solventien geeignet sind.

Ausgangsverbindung VI (R¹⁵ = H) und Nitrier-Reagenz werden zweckmäßigerweise in etwa äquimolaren Mengen eingesetzt; zur Optimierung des Umsatzes an zu nitrierender Verbindung kann es jedoch vorteilhaft sein, das Nitrier-Reagenz im Überschuß zu verwenden, bis etwa zur 10-fachen molaren Menge. Bei der Reaktionsführung ohne Lösungsmittel im Nitrier-Reagenz liegt dieses in einem noch größeren Überschuß vor.

Die Reaktionstemperatur liegt normalerweise bei (- 100) bis 200°C, bevorzugt bei (- 30) bis 50°C.

### E) Synthese von Estern, Amiden, Thioestern und Hydroxamsäureestern I aus Carbonsäuren VI mit R¹⁵ = COOH) auf an sich bekannte Weise:

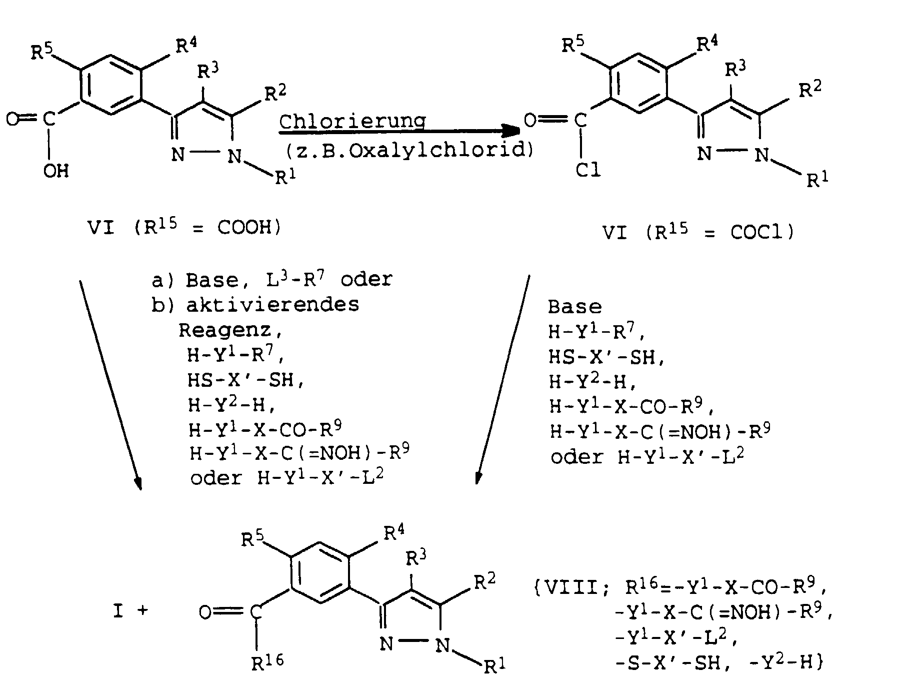

- Y¹: steht für -O-, -S-, -N(R⁸)-, -N(R⁸)-O-;
- Y²: steht für -S-, -N(R⁸)-, -N(R⁸)-O-;
- X': steht für einen definitionsgemäßen Rest X mit Ausnahme von Methylen und substituiertem Methylen;

L² und L³ stehen für übliche Abgangsgruppen wie Halogen, -O-SO₂CH₃, -O-SO₂CF₃, -O-SO₂C₄F₉ oder -O-SO₂-(4-methylphenyl).

Die Reaktionen werden auf an sich bekannte Weise durchgeführt {siehe z.B. Houben-Weyl, Methoden der Organischen Chemie, Georg Thieme Verlag, Stuttgart, Bd. 8, 4. Auflage 1952, S. 471ff., S. 655ff. und S. 686 ff.; Bd. 9, 1955, S. 753ff. und S. 745ff.; Bd. E5, 1985, S. 941ff.}. Beispielsweise können die Carbonsäuren VI mit R¹⁵ = COOH in ihre Säurechloride VII übergeführt werden, aus denen die 5-Pyrazolylbenzoesäure-Derivate I sowie die Verbindungen VIII durch Reaktion mit den entsprechenden Alkoholen, Aminen, Thiolen oder Alkoxyaminen zugänglich sind. Andererseits können die Carbonsäuren VI (R¹⁵ = COOH) auch direkt mit Alkoholen, Aminen, Thiolen oder Alkoxyaminen umgesetzt werden. Hierbei kann die Anwesenheit eines aktivierenden Reagenzes, z.B. N,N'-Dicyclohexylcarbodiimid oder Carbonyldiimidazolid, vorteilhaft sein. Schließlich können Carbonsäuren VI (R¹⁵ = COOH) auch mit Verbindungen R⁷-L³ zu den entsprechenden Estern I (R⁶ = -O-R⁷) alkyliert werden.

Die 3-Phenylpyrazole VIII können anschließend auf an sich bekannte Weise in die Verbindungen I übergeführt werden. So reagieren diejenigen Verbindungen VIII mit R¹⁶ = -Y¹-X-CO-R⁹ mit Hydroxylaminen H₂N-O-Z-R¹⁰ zu Oximen I (R⁶ = -Y¹-X-C(R⁹)=N-O-Z-R¹⁰) (vgl. z.B. Houben-Weyl, Methoden der Organischen Chemie, Georg Thieme Verlag, Stuttgart 1968, Bd. 10/4, S. 10 ff. und Bd. E14b, Stuttart 1990, S. 290 ff.). Oxime, Thiole, Thiosäuren, Säureamide und Hydroxamsäuren VIII (R¹⁶ = -Y¹-X-C(=N-OH)-R⁹, -S-X'-SH und -Y²-H) können durch Alkylierung in Verbindungen I übergeführt werden (vgl. z.B. Houben-Weyl, Methoden der Organischen Chemie, Georg Thieme Verlag, Bd. E5, Stuttgart 1985, S. 934 ff. und S. 1148 ff. sowie Bd. 9, 4. Auflage 1955, S. 749 ff.), beziehungsweise die Thiole VIII (R¹⁶ = -S-Y³-SH) in Thioether VIII (R¹⁶ = -S-X'-S-Z-R¹³), die zu Sulfonen I (R⁶ = -S-X'-SO₂-Z-R¹³) oxidiert werden können (vgl. hierzu z.B. Houben-Weyl, Methoden der Organischen Chemie, Georg Thieme Verlag, Stuttgart, Bd. 9, 4. Auflage 1955, S. 103ff. und S. 227ff.). Weiterhin sind Sulfone I (R⁶ = -Y¹-X-SO₂-Z-R¹³) durch Reaktion der Verbindungen VIII (R¹⁶ = -CO-Y¹-X-L²) mit Thiolen SH-Z-R¹³ und nachfolgender Oxidation zugänglich.

Die Carbonsäuren VI mit R¹⁵ = COOH ihrerseits sind bevorzugt durch Carboxylierung der Halogenverbindungen VI mit R¹⁵ = Chlor, Brom oder Jod auf an sich bekannte Weise (vgl. z.B. JP-A 06/073 015) zugänglich:

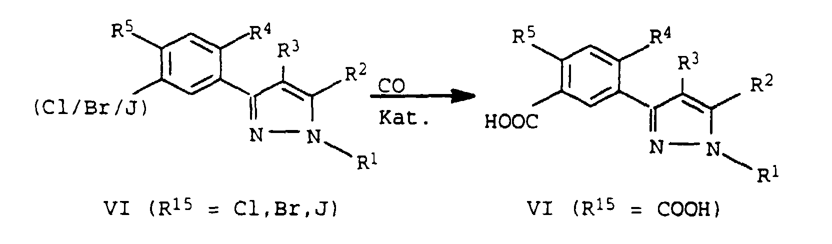

Zweckmäßigerweise arbeitet man in Gegenwart katalytischer Mengen eines Übergangsmetall-Komplexes, vorzugsweise eines Phosphinkomplexes des Palladiums, bei einem Kohlenmonoxid-Druck von etwa 1 bis 100 bar.

Die Reaktionsführung erfolgt in der Regel in einem inerten organischen Lösungsmittel, das mit Wasser mischbar ist, z.B. in Acetonitril oder in Tetrahydrofuran.

Gewöhnlich verwendet man Wasser und eine organische oder anorganische Base wie insbesondere Natriumcarbonat in etwa äquivalenten Mengen oder in einem Überschuß bis etwa zur 10fachen molaren Menge, bezogen auf die Menge an Ausgangsverbindung VI (mit R¹⁵ = Chlor, Brom oder Jod).

Die Reaktionstemperatur liegt üblicherweise zwischen dem Schmelzpunkt des Reaktionsgemisches und etwa 200°C.

Sofern nicht anders angegeben, werden die vorstehend beschriebenen Reaktionen zweckmäßigerweise bei Atmosphärendruck oder unter dem Eigendruck des jeweiligen Reaktionsgemisches durchgeführt.

Die Aufarbeitung der Reaktionsgemische erfolgt in der Regel nach an sich bekannten Methoden, beispielsweise durch Verdünnen der Reaktionslösung mit Wasser und anschließender Isolierung des Produktes mittels Filtration, Kristallisation oder Lösungsmittelextraktion, oder durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die 5-Pyrazolylbenzoesäure-Derivate I können bei der Herstellung als Isomerengemische anfallen, die jedoch gewünschtenfalls nach den hierfür üblichen Methoden wie Kristallisation oder Chromatographie, auch an einem optisch aktiven Adsorbat, in die reinen Isomeren getrennt werden können. Reine optisch aktive Isomere lassen sich vorteilhaft aus entsprechenden optisch aktiven Ausgangsprodukten herstellen.

Landwirtschaftlich brauchbare Salze der Verbindungen I können durch Reaktion mit einer Base des entprechenden Kations, vorzugsweise einem Alkalimetallhydroxid oder -hydrid, oder durch Reaktion mit einer Säure des entprechenden Anions, vorzugsweise der Chlorwasserstoffsäure, Bromwasserstoffsäure, Schwefelsäure, Phosphorsäure oder Salpetersäure, gebildet werden.

Salze von I, deren Metallion kein Alkalimetallion ist, können auch durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris ssp. altissima, Beta vulgaris ssp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus ssp., Manihot esculenta, Medicago sativa, Musa ssp., Nicotiana tabacum (N.rustica), Olea europaea, Oryza sativa, Phaseolus lunatus, Phaseolus vulgaris, Picea abies, Pinus ssp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Des weiteren eignen sich die 5-Pyrazolylbenzoesäure-Derivate I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Ricinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen etwa 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die folgenden Formulierungsbeispiele verdeutlichen die Herstellung solcher Zubereitungen:
I. 20 Gewichtsteile der Verbindung Nr. Ia.003 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. Ia.004 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. Ia.006 werden in einer Mischung gelöst, die aus 25 Gewichtsteilen Cyclohexanon, 65 Gewichtsteilen einer Mineralölfraktion vom Siedepunkt 0 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. Ia.007 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser enthält man eine Spritzbrühe, die 0,1 Gew.-% des Wirkstoffs enthält.
V. 3 Gewichtsteile des Wirkstoffs Nr. Ia.008 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. Ia.009 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. Ia.010 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung Nr. Ia.022 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Ricinusöl) besteht. Man erhält ein stabiles Emulsionskonzentrat.

Die Applikation der Wirkstoffe I bzw. der herbiziden Mittel kann im Vorauflauf- oder im Nachauflaufverfahren erfolgen. Sind die Wirkstoffe fur gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die 5-Pyrazolylbenzoesäure-Derivate I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF3-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### Aufbau des Vorprodukts 5-(4-Chlor-5-difluormethoxy-1-methyl-1Hpyrazol-3-yl)-2,4-dichlorbenzoesäure:

### 1. Stufe: 5-(2,4-Dichlor-5-methylphenyl)-1,2-dihydro-2-methyl-3H-pyrazol-3-on

80,4 g (292 mmol) 2,4-Dichlor-5-methylbenzoylessigsäureethylester wurden in 300 ml Diethylenglycoldimethylether langsam mit 13,5 g (292 mmol) Methylhydrazin versetzt. Nach 4 Stunden bei 100°C wurde die Lösung in 1 l Eiswasser eingerührt. Der gebildete Niederschlag wurde abfiltriert, mit wenig Methylenchlorid gewaschen und getrocknet. Ausbeute: 38,8 g.
¹H-NMR (270 MHz, in d⁶-Dimethylsulfoxid): δ [ppm] = 2,32 (s,3H), 3,60 (s,3H), 5,89 (s,1H), 7,58 (s,1H), 7,76 (s,1H), 11,12 (s,1H).

### 2. Stufe: 3-(2,4-Dichlor-5-methylphenyl)-5-difluor-methoxy-1-methyl-1H-pyrazol

Zu einer Lösung von 37 g (144 mmol) 5-(2,4-Dichlor-5-methylphenyl)-1,2-dihydro-2-methyl-3H-pyrazol-3-on in 390 ml Dioxan wurden 28,8 g (720 mmol) Natriumhydroxid, gelöst in 240 ml Wasser, gegeben. Anschließend leitete man bei 60 - 65°C im Verlauf von 4 Stunden Chlordifluormethan ein, wonach die Reaktionslösung in 1 l Wasser eingerührt wurde. Danach extrahierte man mit Methyl-tert.-butylether. Die organische Phase wurde über Magnesiumsulfat getrocknet, filtriert und dann eingeengt. Die Reinigung des Rohproduktes erfolgte mittels Säulenchromatographie an Kieselgel (Eluent: Diethylether). Ausbeute: 35,1 g.
¹H-NMR (270 MHz, in CDCl₃): δ [ppm] = 2,39 (s,3H), 3,84 (s,3H), 6,45 (s,1H), 6,59 (t,1H), 7,46 (s,1H), 7,70 (s,1H).

### 3. Stufe: 4-Chlor-3-(2,4-dichlor-5-methylphenyl)-5-difluor-methoxy-1-methyl-1H-pyrazol

Zu einer Lösung von 35 g (114 mmol) 3-(2,4-Dichlor-5-methylphenyl)-5-difluormethoxy-1-methyl-1H-pyrazol in 120 ml Tetrachlormethan wurden 16,9 g (125 mmol) Sulfurylchlorid getropft. Nach 2 Stunden Rühren wurde die Reaktionsmischung mit Wasser, gesättigter wässriger Natriumhydrogencarbonat-Lösung und gesättigter wässriger Natriumchlorid-Lösung gewaschen, dann über Magnesiumsulfat getrocknet, filtriert und eingeengt.
Ausbeute: 30,8 g.
¹H-NMR (270 MHz, in CDCl₃): δ [ppm] = 2,39 (s,3H), 3,85 (s,3H), 6,74 (t,1H), 7,30 (s,1H), 7,49 (s,1H).

### 4. Stufe: 3- (5-Dibrommethyl-2,4-dichlor-phenyl)-4-chlor-5-difluormethoxy-1-methyl-1H-pyrazol

Zu einer Lösung von 28 g (82 mmol) 4-Chlor-3-(2,4-dichlor-5-methylphenyl)-5-difluormethoxy-1-methyl-1H-pyrazol in 1,5 1 Tetrachlormethan wurden 99,3 g (560 mmol) N-Bromsuccinimid gegeben. Dann bestrahlte man das Reaktionsgemisch 2 Stunden lang mit einer UV-Lampe und einer 150 W Quecksilberhochdrucklampe. Anschließend wurde der Feststoffanteil abfiltriert, wonach man das Filtrat einengte. Ausbeute: quantitativ.
¹H-NMR (270 MHz, in CDCl₃): δ [ppm] = 3,88 (s,3H), 6,75 (t,1H), 7,05 (s,1H), 7,50 (s,1H), 8,08 (s,1H).

### 5. Stufe: 5-(4-Chlor-5-difluormethoxy-1-methyl-1H-pyrazol-3-yl)-2,4-dichlorbenzaldehyd

Zu 65 ml konzentrierter Schwefelsäure wurden bei 85°C portionsweise 45,5 g (91 mmol) 3-(5-Dibrommethyl-2,4-dichlorphenyl)-4-chlor-5-difluormethoxy-1-methyl-1H-pyrazol gegeben, wobei man langsam auf 95°C erwärmte. Anschließend wurde bei 103°C noch 5 Minuten gerührt, wonach man das Reaktionsgemisch in 600 ml Eiswasser einrührte. Das Produkt wurde mit Dichlormethan extrahiert. Die organische Phase wurde mit gesättigter Natriumchloridlösung gewaschen, über Magnesiumsulfat getrocknet, filtriert und schließlich eingeengt. Die Reinigung des Rohprodukts erfolgte durch Lösen in Hexan/Ethylacetat (1:1) und filtrieren der Lösung über ein Bett aus Kieselgel. Ausbeute nach Einengen: 20 g.
¹H-NMR (270 MHz, in CDCl₃): δ [ppm] = 3,87 (s,3H), 6,77 (t,1H), 7,65 (s,1H), 8,02 (s,1H), 10,41 (s,1H).

### 6. Stufe: 5-(4-Chlor-5-difluormethoxy-1-methyl-1H-pyrazol-3-yl)-2,4-dichlorbenzoesäure

Zu einer Lösung von 21,3 g (60 mmol) 5-(4-Chlor-5-difluormethoxy-1-methyl-1H-pyrazol-3-yl)-2,4-dichlorbenzaldehyd in 120 ml Acetonitril wurde bei 10-15°C eine Lösung von 2,5 g (16 mmol) Natriumdihydrogenphosphat-Dihydrat in 25 ml Wasser getropft. In diese Mischung tropfte man 6 ml einer 30 gew.-%igen Wasserstoffperoxid-Lösung und anschließend während zwei Stunden eine Lösung von 8,7 g (96 mmol) Natriumchlorit in 80 ml Wasser. Das Reaktionsgemisch wurde noch eine Stunde gerührt und dann mit 3N Salzsäure angesäuert. Aus der erhaltenen Suspension wurde der Feststoffanteil abgetrennt und getrocknet. Ausbeute: 13,7 g;
Fp. 159-160°C.

### Beispiel 2

### 5-(4-Chlor-5-difluormethoxy-1-methyl-1H-pyrazol-3-yl)-2,4-dichlorbenzoesäure-2-(ethoxyimino)ethylester (Nr. Ia.003)

Eine Lösung von 1,9 g (5 mmol) 5-(4-Chlor-5-difluormethoxy-1-methyl-1H-pyrazol-3-yl)-2,4-dichlor-benzoesäure, 0,9 g (6,5 mmol) Kaliumcarbonat (gemahlen) und 0,6 g (5 mmol) O-Ethyl-chloracetaldehydoxim in 50 ml Dimethylformamid wurde 3 Stunden bei 50-60°C gerührt, wonach man einengte. Der Rückstand wurde in Ethylacetat und Wasser aufgenommen. Anschließend trennte man die wässrige Phase ab und mischte sie nochmals kurz mit Ethylacetat. Die vereinigten organischen Phasen wurden über Magnesiumsulfat getrocknet, dann filtriert und eingeengt. Die Reinigung des Rohprodukts erfolgte mittels Säulenchromatographie an Kieselgel (Eluent: Hexan/Ethylacetat = 4:1). Ausbeute: 1,6 g.
¹H-NMR (250 MHz, in CDCl₃): δ [ppm] = 1,26 (m,3H), 3,85 (s,3H), 4,10-4,23 (m,2H), 4,90 und 5,12 (2d, zusammen 2H), 6,71 (t,1H), 6,88 und 7,54 (2t, zusammen 1H), 7,62 (m,1H), 8,02 (m,1H).

### Beispiel 3

### 5-(4-Chlor-5-difluormethoxy-1-methyl-1H-pyrazol-3-yl)-2,4-dichlorbenzoesäure-2-(2-pyrrolidon-1-yl)-ethylester (Nr. Ia.331)

Eine Lösung von 1 g (2,6 mmol) 5-(4-Chlor-5-difluormethoxy-1-methyl-1H-pyrazol-3-yl)-2,4-dichlorbenzoesäurechlorid und 0,5 g (3,8 mmol) 2-(2-Pyrrolidon-1-yl)ethanol in 20 ml Pyridin wurde etwa 15 Stunden gerührt, wonach man das Reaktionsgemisch einengte. Der Rückstand wurde mit 10 ml Wasser und 20 ml Ethylacetat versetzt. Dann trennte man die organische Phase ab, trocknete sie über Magnesiumsulfat und engte ein. Die Reinigung des Rohprodukts erfolgte mittels Säulenchromatographie an Kieselgel (Laufmittel: zunächst Hexan/Ethylacetat = 1:1, dann Ethylacetat). Ausbeute: 0,8 g.

### Vorstufe:

### 5-(4-chlor-5-difluormethoxy-1-methyl-1H-pyrazol-3-yl)-2,4-dichlorbenzoesäurechlorid

Zu einer Lösung von 7,7 g (20 mmol) 5-(4-Chlor-5-difluormethoxy-1-methyl-1H-pyrazol-3-yl)-2,4-dichlor-benzoesäure in 300 ml Toluol wurden 20,5 g (160 mmol) Oxalylchlorid getropft. Nach 6 Stunden Rühren bei Rückflußtemperatur wurde das Reaktionsgemisch eingeengt. Ausbeute: quantitativ.
¹H-NMR (250 MHz, in CDCl₃): δ [ppm] = 3,87 (s,3H), 6,72 (t,1H), 7,66 (s,1H), 8,21 (s,1H).

### Beispiel 4

### 5-(4-Chlor-5-difluormethoxy-1-methyl-1H-pyrazol-3-yl)-2,4-dichlorbenzoesäure-2-(allyloxyimino)ethylester (Nr. Ia.005)

Eine Lösung von 2 g (5 mmol) 5-(4-Chlor-5-difluormethoxy-1-methyl-1H-pyrazol-3-yl)-2,4-dichlor-benzoesäurechlorid und 0,6 g (5 mmol) O-Allyl-glycolaldehydoxim in 40 ml Pyridin wurde 5 Stunden auf 50-60°C erwärmt, wonach man einengte. Der Rückstand wurde in Ethylacetat aufgenommen. Die erhaltene Lösung wurde mit 2 N Salzsäure und gesättigter Kochsalzlösung gewaschen, über Magnesiumsulfat getrocknet, filtriert und dann eingeengt. Die Reinigung des Rohprodukts erfolgte mittels Säulenchromatographie an Kieselgel (Eluent: Hexan/Ethylacetat = 1:1). Ausbeute: 1,2 g.
¹H-NMR (250 MHz, in CDCl₃): δ [ppm] = 3,84 (s,3H), 4,55-4,66 (m,2H), 4,91 und 5,12 (2d, zusammen 2H), 5,20-5,37 (m,2H), 5,88-6,08 (m,1H), 6,71 (t,1H), 6,92 und 7,60 (2t, zusammen 1H), 7,64 (m,1H), 8,00 (m,1H).

### Beispiel 5

### 5-(4-Chlor-5-difluormethoxy-1-methyl-1H-pyrazol-3-yl)-2,4-dichlorbenzoesäure-[N-[2-(1,3-dioxolan-2-yl)ethyl]-N-methyl]amid (Nr. Ia.458)

Zu einer Lösung von 1 g (2,7 mmol) 5-(4-Chlor-5-difluormethoxy-1-methyl-1H-pyrazol-3-yl)-2,4-dichlorbenzoesäure und 0,66 g (4,1 mmol) 1,1-Carbonyldiimidazol in 20 ml Tetrahydrofuran wurden nach 1 Stunde rühren 0,55 g (4,1 mmol) N-[2-(1,3-Dioxolan-2-yl)-ethyl]-N-methyl-amin gegeben. Anschließend rührte man weitere 16 Stunden bei etwa 20°C. Dann wurde das Reaktionsgemisch eingeengt. Die Reinigung des Rohprodukts erfolgte chromatographisch an Kieselgel (Laufmittel: Hexan/Ethylacetat = 1:1). Ausbeute: 0,4 g.

In den folgenden Tabellen 2 und 3 sind neben den vorstehend beschriebenen Verbindungen noch weitere 5-Pyrazolylbenzoesäure-Derivate I aufgeführt, die in analoger Weise hergestellt wurden oder herstellbar sind:

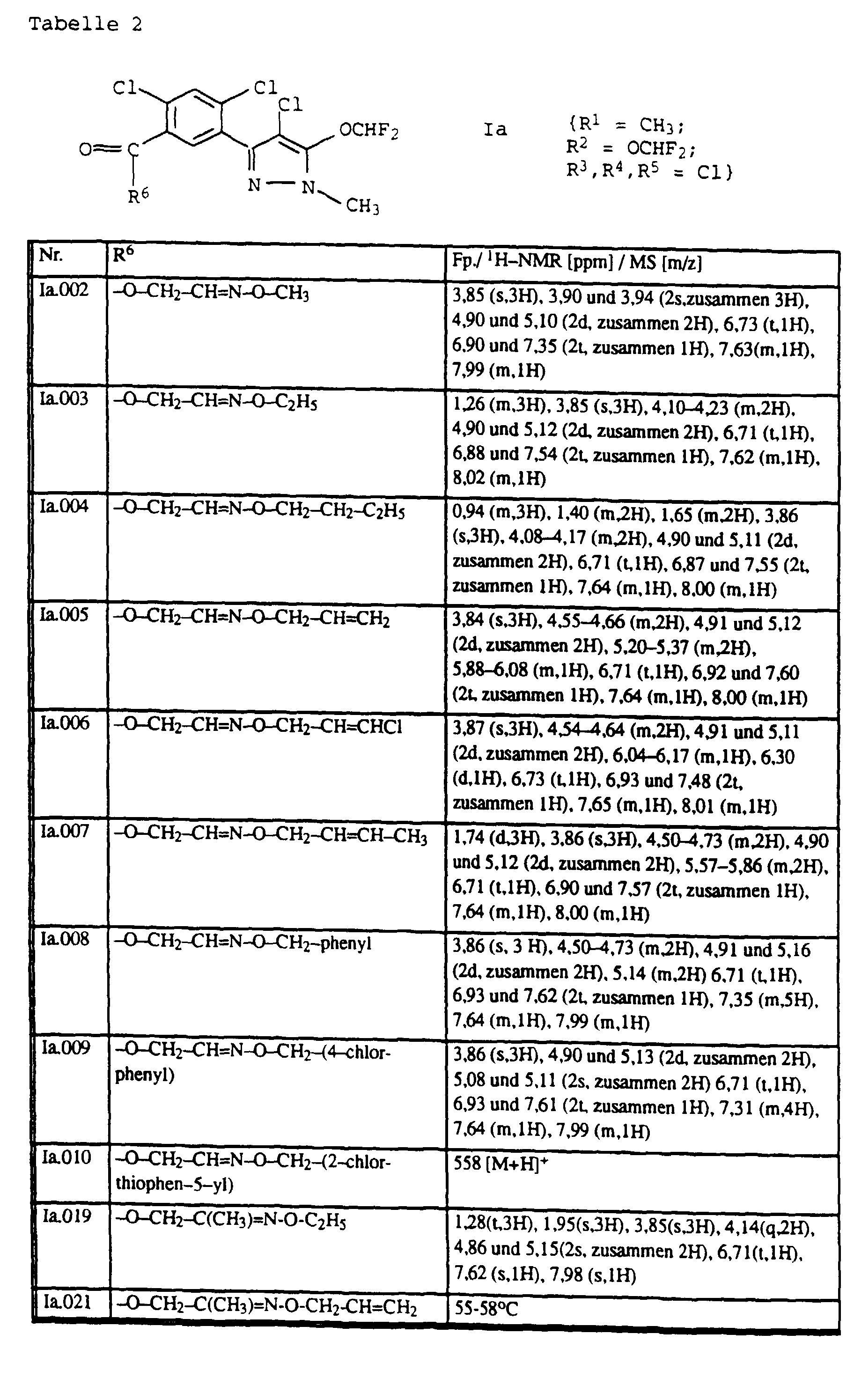

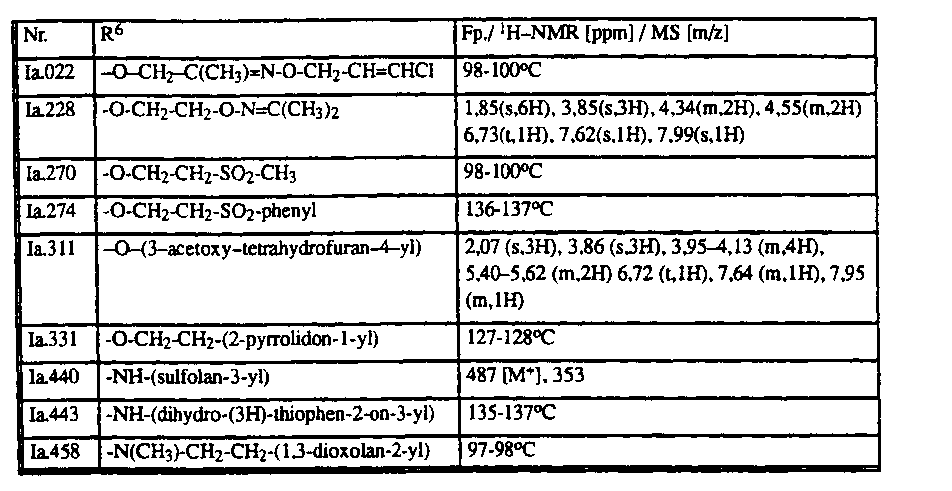

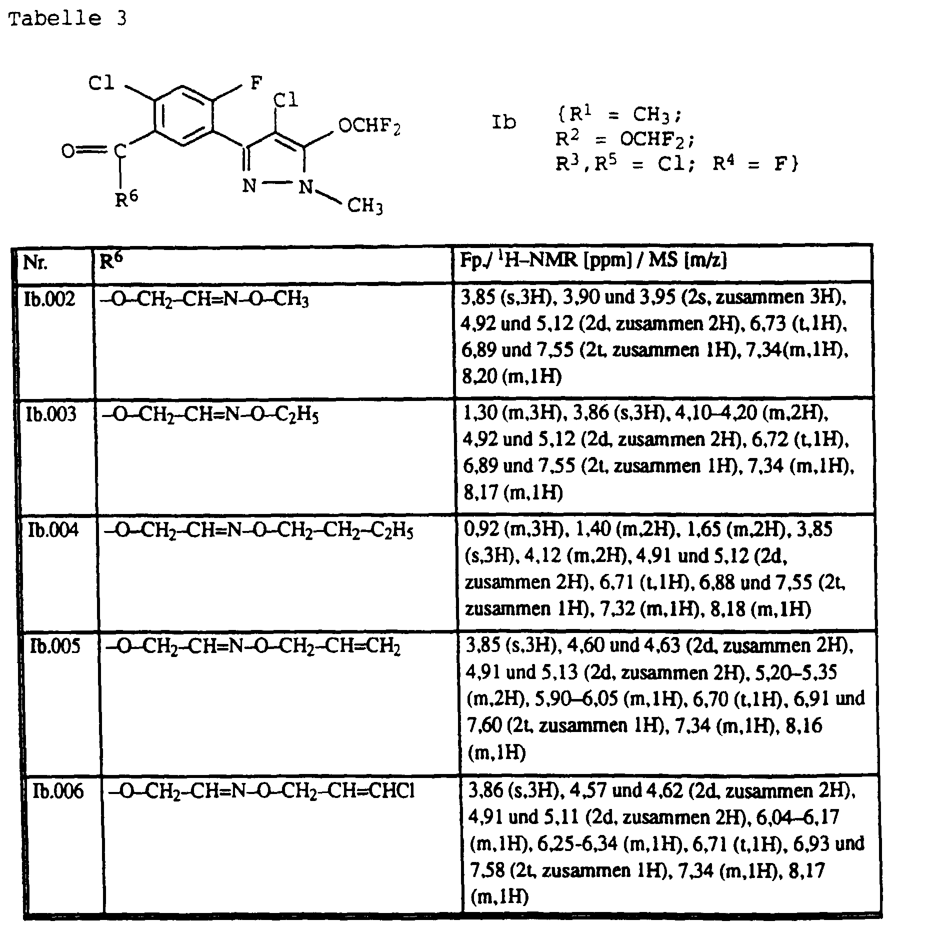

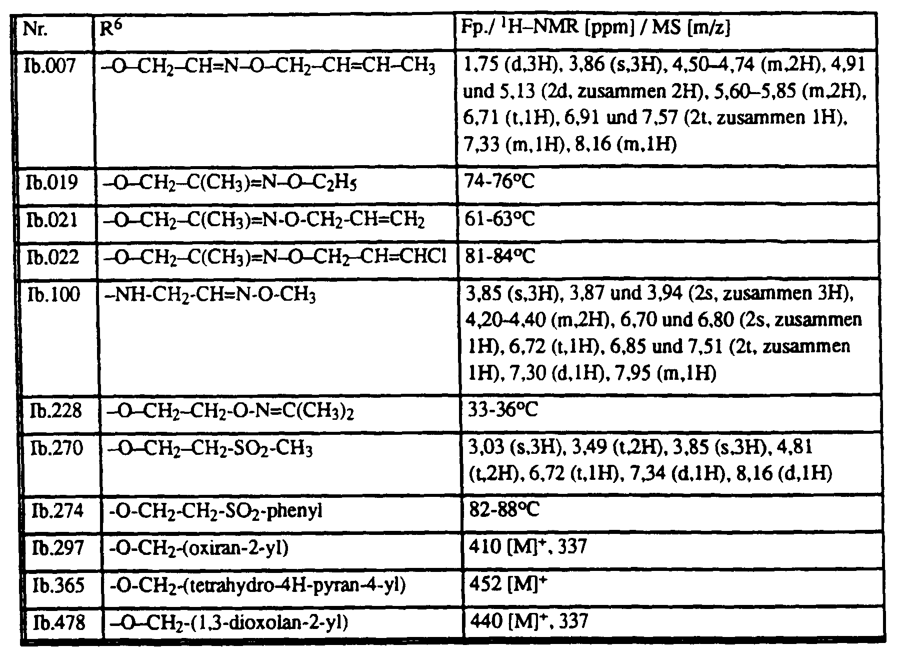

### Anwendungsbeispiele (herbizide Wirksamkeit)

Die herbizide Wirkung der 5-Pyrazolylbenzoesäure-Derivate I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,0156 oder 0,0078 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| **Lateinischer Name** | **Deutscher Name** | **Englischer Name** |
|---|---|---|
| **Amaranthus retroflexus** | **Zurückgekrümmter Fuchsschwanz** | **Redroot pigweed** |
| **Galium aparine** | **Klettenlabkraut** | **Catchweed bedstraw** |
| **Polygonum persicaria** | **Flohknöterich** | **Lady's thumb** |
| **Solanum nigrum** | **Schwarzer Nachtschatten** | **Black nightshade** |
| **Veronica subspecies** | **Ehrenpreisarten** | **Speedwell** |

Bei Aufwandmengen von 0,0156 und 0,0078 kg/ha a.S. zeigte die Verbindung Nr. Ia.003 im Nachauflaufverfahren eine sehr gute Wirkung gegen die o.g. Pflanzen.

### Anwendungsbeispiele (desikkative/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag/Nachttemperatur = 27/20°C).

Die jungen Baumwollpflanzen wurden tropfnaß mit wäßrigen Aufbereitungen der Wirkstoffe (unter Zusatz von 0,15 Gew.-% des tensidisch wirksamen Fettalkoholalkoxylats Plurafac® LF 700, bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.

Bei den unbehandelten Kontrollpflanzen trat kein Blattfall auf.

## Patentansprüche

1. 5-Pyrazolylbenzoesäure-Derivate der Formel I 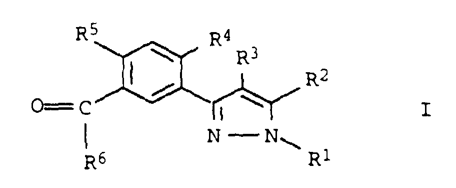 in der die Substituenten folgende Bedeutungen haben:
R¹ Wasserstoff, C₁-C₄-Alkyl, Cyano-C₁-C₄-alkyl oder C₁-C₄-Halogenalkyl;
R² C₁-C₄-Alkoxy, C₁-C₄-Alkylthio, C₁-C₄-Halogenalkoxy oder C₁-C₄-Halogenalkylthio;
R³ Wasserstoff, Cyano, Nitro, Halogen;
R⁴ Halogen;
R⁵ Cyano, Halogen, C₁-C₄-Alkyl, C₁-C₄-Halogenalkyl, C₁-C₄-Alkoxy oder C₁-C₄-Halogenalkoxy;
R⁶ -O-R⁷, -S-R⁷, -N(R⁸)-R⁷ oder -N(R⁸)-O-R⁷;
R⁷ steht für -X-C(R⁹)=N-O-R¹⁰, -X-C(R⁹)=N-O-Z-R¹⁰, -X-O-N=C(R¹¹,R¹²), -X-SO₂-R¹³ oder für gesättigtes 3- bis 7-gliedriges Heterocyclyl oder Heterocyclyl-C₁-C₄-alkyl, wobei der Heterocyclus neben Methylengliedern ein bis drei Ringglieder enthält, ausgewählt aus der Gruppe bestehend aus drei Azabrücken und zwei Sauerstoff- oder Schwefelatomen, und wobei gewünschtenfalls ein oder zwei Methylengruppen des Heterocyclus durch Carbonyl, Thiocarbonyl und/oder Sulfonyl ersetzt sein können, wobei der Heterocyclus unsubstituiert sein oder ein bis vier Substituenten tragen kann, jeweils ausgewählt aus der Gruppe bestehend aus Halogen, C₁-C₄-Alkyl, C₁-C₄-Alkoxy, (C₁-C₄-Alkoxy)carbonyl, (C₁-C₄-Alkyl)carbonyl, (C₁-C₄-Alkyl)carbonyloxy und (C₁-C₄-Alkyl)carbonylamino;
R⁸ steht für Wasserstoff, C₁-C₄-Alkyl, C₁-C₄-Halogenalkyl, C₃-C₅-Alkenyl, C₃-C₅-Halogenalkenyl, C₃-C₅-Alkinyl oder C₁-C₄-Alkylsulfonyl;
R⁹ steht für Wasserstoff, Cyano, C₁-C₄-Alkyl, C₁-C₄-Alkoxy oder C₁-C₄-Alkylthio;
R¹⁰ steht für Wasserstoff, C₁-C₄-Alkyl, C₁-C₄-Halogenalkyl, C₁-C₄-Cyanoalkyl, Di-(C₁-C₄-alkyl)amino-C₁-C₄-alkyl, C₁-C₄-Alkoxy-C₁-C₄-alkyl, C₁-C₄-Alkylthio-C₁-C₄-alkyl, (C₁-C₄-Alkyl)carbonyl, (C₁-C₄-Alkoxy)carbonyl, Di-(C₁-C₄-alkyl)aminocarbonyl, (C₁-C₄-Halogenalkyl)carbonyl, C₃-C₅-Alkenyl, C₃-C₅-Halogenalkenyl, Di-(C₁-C₄-alkyl)amino-C₃-C₅-alkenyl, C₁-C₄-Alkoxy-C₃-C₅-alkenyl, C₁-C₄-Alkylthio-C₃-C₅-alkenyl, C₃-C₅-Alkinyl, C₃-C₅-Halogenalkinyl, C₃-C₇-Cycloalkyl, das gewünschtenfalls ein bis drei C₁-C₃-Alkylreste tragen kann,
oder für Phenyl, Benzoyl oder 5- oder 6-gliedriges Heteroaryl, das ein bis drei Heteroatome enthält, ausgewählt aus der Gruppe bestehend aus drei Stickstoffatomen und einem Sauerstoff- oder Schwefelatom, wobei die Phenyl- und Heteroarylringe unsubstituiert sein oder an jedem substituierbaren Ringglied einen der folgenden Substituenten tragen können: Nitro, Cyano, Halogen, C₁-C₄-Alkyl, C₁-C₄-Halogenalkyl oder C₁-C₄-Alkoxy;
R¹¹, R¹² stehen unabhängig voneinander für Wasserstoff, C₁-C₄-Alkyl, C₁-C₄-Halogenalkyl, C₁-C₄-Alkoxy-C₁-C₄-alkyl, C₃-C₅-Alkenyl, C₃-C₅-Halogenalkenyl, C₃-C₅-Alkinyl, C₃-C₅-Halogenalkinyl, C₃-C₇-Cycloalkyl, das gewünschtenfalls ein bis drei C₁-C₃-Alkylreste tragen kann, oder für Phenyl, das gewünschtenfalls an jedem substituierbaren Ringglied einen der folgenden Substituenten tragen kann: Nitro, Cyano, Halogen, C₁-C₄-Alkyl, C₁-C₄-Halogenalkyl oder C₁-C₄-Alkoxy,
oder R¹¹ und R¹² bilden zusammen mit dem gemeinsamen Kohlenstoffatom, an das sie gebunden sind, einen gesättigten 3- bis 8-gliedrigen Ring, der neben Methylengliedern gewünschtenfalls ein oder zwei Sauerstoff-, Schwefel- und/oder Azaringglieder enthalten kann, wobei der Ring unsubstituiert sein oder ein bis vier C₁-C₄-Alkylreste tragen kann;
R¹³ steht für C₁-C₄-Alkyl, C₁-C₄-Halogenalkyl, C₁-C₄-Cyanoalkyl, Di-(C₁-C₄-alkyl)amino-(C₁-C₄-alkyl), C₁-C₄-Alkoxy-C₁-C₄-alkyl, C₁-C₄-Alkylthio-C₁-C₄-alkyl, C₃-C₅-Alkenyl, C₃-C₅-Halogenalkenyl, Di-(C₁-C₄-alkyl)amino-C₃-C₅-alkenyl, C₁-C₄-Alkoxy-C₃-C₅-alkenyl, C₁-C₄-Alkylthio-C₃-C₅-alkenyl, C₃-C₅-Alkinyl, C₃-C₅-Halogenalkinyl, C₃-C₇-Cycloalkyl, das gewünschtenfalls ein bis drei C₁-C₃-Alkylreste tragen kann, für Phenyl oder für 5- oder 6-gliedriges Heteroaryl, das ein bis drei Heteroatome enthält, ausgewählt aus der Gruppe bestehend aus drei Stickstoffatomen und einem Sauerstoff- oder Schwefelatom, wobei der Phenyl- und Heteroarylring unsubstituiert sein oder an jedem substituierbaren Ringglied einen der folgenden Substituenten tragen kann: Nitro, Cyano, Halogen, C₁-C₄-Alkyl, C₁-C₄-Halogenalkyl oder C₁-C₄-Alkoxy;
X und Z stehen unabhängig voneinander für C₁-C₄-Alkylenketten, die unsubstituiert sein oder ein bis vier Substituenten tragen können, jeweils ausgewählt aus der Gruppe bestehend aus Halogen, C₁-C₄-Alkyl, C₁-C₄-Halogenalkyl und C₁-C₄-Alkoxy;
sowie die landwirtschaftlich brauchbaren Salze der Verbindungen I.

2. Verwendung der 5-Pyrazolylbenzoesäure-Derivate I und ihrer landwirtschaftlich brauchbaren Salze, gemäß Anspruch 1, als Herbizide oder zur Desikkation/Defoliation von Pflanzen.

3. Herbizides Mittel, enthaltend eine herbizid wirksame Menge mindestens eines 5-Pyrazolylbenzoesäure-Derivates der Formel I oder eines landwirtschaftlich brauchbaren Salzes von I, gemaß Anspruch 1, und mindestens einen inerten flüssigen und/oder festen Trägerstoff sowie gewünschtenfalls mindestens einen oberflachenaktiven Stoff.

4. Mittel zur Desikkation und/oder Defoliation von Pflanzen, enthaltend eine desikkant und/oder defoliant wirksame Menge mindestens eines 5-Pyrazolylbenzoesäure-Derivates der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

5. Verfahren zur Herstellung von herbizid wirksamen Mitteln, dadurch gekennzeichnet, daß man eine herbizid wirksame Menge mindestens eines 5-Pyrazolylbenzoesäure-Derivates der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

6. Verfahren zur Herstellung von desikkant und/oder defoliant wirksamen Mitteln, dadurch gekennzeichnet, daß man eine desikkant und/oder defoliant wirksame Menge mindestens eines 5-Pyrazolylbenzoesäure-Derivates der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

7. Verfahren zur Bekämpfung von unerwünschtem Pflanzenwuchs, dadurch gekennzeichnet, daß man eine herbizid wirksame Menge mindestens eines 5-Pyrazolylbenzoesäure-Derivates der Formel I oder eines landwirtschaftlich brauchbaren Salzes von I, gemäß Anspruch 1, auf Pflanzen, deren Lebensraum oder auf Saatgut einwirken läßt.

8. Verfahren zur Desikkation und/oder Defoliation von Pflanzen, dadurch gekennzeichnet, daß man eine desikkant und/oder defoliant wirksame Menge mindestens eines 5-Pyrazolylbenzoesäure-Derivates der Formel I oder eines landwirtschaftlich brauchbaren Salzes von I, gemäß Anspruch 1, auf Pflanzen einwirken läßt.

## Claims

1. A 5-pyrazolylbenzoic acid derivative of the formula I 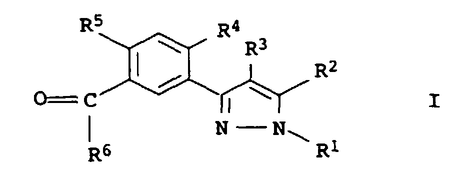 where the substituents have the following meanings:
R¹ is hydrogen, C₁-C₄-alkyl, cyano-C₁-C₄-alkyl or C₁-C₄-haloalkyl;
R² is C₁-C₄-alkoxy, C₁-C₄-alkylthio, C₁-C₄-haloalkoxy or C₁-C₄-haloalkylthio;
R³ is hydrogen, cyano, nitro, halogen;
R⁴ is halogen;
R⁵ is cyano, halogen, C₁-C₄-alkyl, C₁-C₄-haloalkyl, C₁-C₄-alkoxy or C₁-C₄-haloalkoxy;
R⁶ is -O-R⁷, -S-R⁷, -N(R⁸)-R⁷ or -N(R⁸)-O-R⁷;
R⁷ is -X-C(R⁹)=N-O-R¹⁰, -X-C(R⁹)=N-O-Z-R¹⁰, -X-O-N=C(R¹¹,R¹²), -X-XO₂-R¹³ or saturated 3- to 7-membered heterocyclyl or heterocyclyl-C₁-C₄-alkyl, the heterocycle containing, besides methylene members, one to three ring members selected from the group consisting of three aza bridges and two oxygen or sulfur atoms, and it being possible, if desired, for one or two methylene groups of the heterocycle to be replaced by carbonyl, thiocarbonyl and/or sulfonyl, it being possible for the heterocycle to be unsubstituted or to have attached to it one to four substituents in each case selected from the group consisting of halogen, C₁-C₄-alkyl, C₁-C₄-alkoxy, (C₁-C₄-alkoxy)carbonyl, (C₁-C₄-alkyl)carbonyl, (C₁-C₄-alkyl)carbonyloxy and (C₁-C₄-alkyl)carbonylamino;
R⁸ is hydrogen, C₁-C₄-alkyl, C₁-C₄-haloalkyl, C₃-C₅-alkenyl, C₃-C₅-haloalkenyl, C₃-C₅-alkynyl or C₁-C₄-alkylsulfonyl;
R⁹ is hydrogen, cyano, C₁-C₄-alkyl, C₁-C₄-alkoxy or C₁-C₄-alkylthio;
R¹⁰ is hydrogen, C₁-C₄-alkyl, C₁-C₄-haloalkyl, C₁-C₄-cyanoalkyl, di(C₁-C₄-alkyl)amino-C₁-C₄-alkyl, C₁-C₄-alkoxy-C₁-C₄-alkyl, C₁-C₄-alkylthio-C₁-C₄-alkyl, (C₁-C₄-alkyl)carbonyl, (C₁-C₄-alkoxy)carbonyl, di(C₁-C₄-alkyl)aminocarbonyl, (C₁-C₄-haloalkyl)carbonyl, C₃-C₅-alkenyl, C₃-C₅-haloalkenyl, di(C₁-C₄-alkyl)amino-C₃-C₅-alkenyl, C₁-C₄-alkoxy-C₃-C₅-alkenyl, C₁-C₄-alkylthio-C₃-C₅-alkenyl, C₃-C₅-alkynyl, C₃-C₅-haloalkynyl, C₃-C₇-cycloalkyl which, if desired, can have attached to it one to three C₁-C₃-alkyl radicals,
or is phenyl, benzoyl or 5- or 6-membered heteroaryl which contains one to three hetero atoms selected from the group consisting of three nitrogen atoms and one oxygen or sulfur atom, it being possible for the phenyl and heteroaryl rings to be unsubstituted or to have attached to each substitutable ring member one of the following substituents: nitro, cyano, halogen, C₁-C₄-alkyl, C₁-C₄-haloalkyl or C₁-C₄-alkoxy;
R¹¹, R¹² independently of one another are hydrogen, C₁-C₄-alkyl, C₁-C₄-haloalkyl, C₁-C₄-alkoxy-C₁-C₄-alkyl, C₃-C₅-alkenyl, C₃-C₅-haloalkenyl, C₃-C₅-alkynyl, C₃-C₅-haloalkynyl, C₃-C₇-cycloalkyl, which, if desired, can have attached to it one to three C₁-C₃-alkyl radicals, or are phenyl which, if desired, can have attached to each substitutable ring member one of the following substituents: nitro, cyano, halogen, C₁-C₄-alkyl, C₁-C₄-haloalkyl or C₁-C₄-alkoxy,
or R¹¹ and R¹² together with the joint carbon atom to which they are bonded form a saturated 3- to 8-membered ring which, if desired, can contain, besides methylene members, one or two oxygen, sulfur and/or aza ring members, it being possible for the ring to be unsubstituted or to have attached to it one to four C₁-C₄-alkyl radicals;
R¹³ is C₁-C₄-alkyl, C₁-C₄-haloalkyl, C₁-C₄-cyanoalkyl, di(C₁-C₄-alkyl)amino-(C₁-C₄-alkyl), C₁-C₄-alkoxy-C₁-C₄-alkyl, C₁-C₄-alkylthio-C₁-C₄-alkyl, C₃-C₅-alkenyl, C₃-C₅-haloalkenyl, di(C₁-C₄-alkyl)amino-C₃-C₅-alkenyl, C₁-C₄-alkoxy-C₃-C₅-alkenyl, C₁-C₄-alkylthio-C₃-C₅-alkenyl, C₃-C₅-alkynyl, C₃-C₅-haloalkynyl, C₃-C₇-cycloalkyl which, if desired, can have attached to it one to three C₁-C₃-alkyl radicals, or is phenyl, or is 5- or 6-membered heteroaryl which contains one to three hetero atoms selected from the group consisting of three nitrogen atoms and one oxygen or sulfur atom, it being possible for the phenyl and heteroaryl ring to be unsubstituted or to have attached to each substitutable ring member one of the following substituents: nitro, cyano, halogen, C₁-C₄-alkyl, C₁-C₄-haloalkyl or C₁-C₄-alkoxy;
X and Z independently of one another are C₁-C₄-alkylene chains which can be unsubstituted or can have attached to them one to four substituents in each case selected from the group consisting of halogen, C₁-C₄-alkyl, C₁-C₄-haloalkyl and C₁-C₄-alkoxy;
or an agriculturally useful salt of a compound I.

2. The use of the 5-pyrazolylbenzoic acid derivatives I and of their agriculturally useful salts as claimed in claim 1 as herbicides or for desiccating/defoliating of plants.

3. A herbicidal composition comprising a herbicidally active amount of at least one 5-pyrazolylbenzoic acid derivative of the formula I or at least one agriculturally useful salt of I as claimed in claim 1, and at least one inert liquid and/or solid carrier and, if desired, at least one surfactant.

4. A composition for desiccating and/or defoliating plants comprising such an amount of at least one 5-pyrazolylbenzoic acid derivative of the formula I or at least one agriculturally useful salt of I as claimed in claim 1 that it acts as a desiccant and/or defoliant, and at least one inert liquid and/or solid carrier and, if desired, at least one surfactant.

5. A process for the preparation of herbicidally active compositions, which comprises mixing a herbicidally active amount of at least one 5-pyrazolylbenzoic acid derivative of the formula I or at least one agriculturally useful salt of I as claimed in claim 1, and at least one inert liquid and/or solid carrier and, if desired, at least one surfactant.

6. A process for the preparation of compositions which act as desiccants and/or defoliants, which comprises mixing such an amount of at least one 5-pyrazolylbenzoic acid derivative of the formula I or at least one agriculturally useful salt of I as claimed in claim 1 that it acts as a desiccant and/or defoliant, and at least one inert liquid and/or solid carrier and, if desired, at least one surfactant.

7. A method of controlling undesirable vegetation, which comprises allowing a herbicidally active amount of at least one 5-pyrazolylbenzoic acid derivative of the formula I or at least one agriculturally useful salt of I as claimed in claim 1 to act on plants, their environment or seed.

8. A method of desiccating and/or defoliating plants, which comprises allowing such an amount of at least one 5-pyrazolylbenzoic acid derivative of the formula I or at least one agriculturally useful salt of I as claimed in claim 1 that it acts as a desiccant and/or defoliant to act on plants.

## Revendications

1. Dérivés de l'acide 5-pyrazolylbenzoïque répondant à la formule I 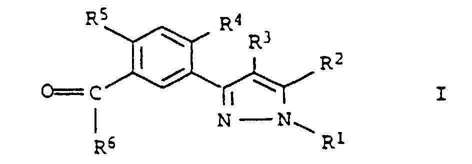 dans laquelle les symboles ont les significations suivantes :
R¹ : l'hydrogène, un groupe alkyle en C1-C4, cyano-alkyle en C1-C4 ou halogénoalkyle en C1-C4 ;
R² : un groupe alcoxy en C1-C4, alkylthio en C1-C4, halogénoalcoxy en C1-C4 ou halogénoalkylthio en C1-C4 ;
R³ : l'hydrogène, un groupe cyano, nitro, un halogène ;
R⁴ : un halogène ;
R⁵ : un groupe cyano, un halogène, un groupe alkyle en C1-C4, halogénoalkyle en C1-C4, alcoxy en C1-C4 ou halogénoalcoxy en C1-C4 ;
R⁶ : -O-R⁷, -S-R⁷, -N(R⁸)-R⁷ ou -N(R⁸)-O-R⁷ ;
R⁷ : -X-C(R⁹)=N-O-R¹⁰, -X-C(R⁹)=N-O-Z-R¹⁰, -X-O-N=C(R¹¹, R¹²), -X-SO₂-R¹³ ou un groupe hétérocycle ou hétérocyclyl-alkyle en C1-C4 saturé, de trois à sept chaînons, l'hétérocycle contenant, en plus des chaînons méthylène, un à trois chaînons cycliques choisis dans le groupe consistant en trois ponts aza et deux atomes d'oxygène ou de soufre, un ou deux groupes méthylène de l'hétérocycle pouvant éventuellement être remplacés par des groupes carbonyle, thiocarbonyle et/ou sulfonyle, l'hétérocycle étant non substitué ou pouvant porter un à quatre substituants choisis chacun parmi les halogènes, les groupes alkyle en C1-C4, alcoxy en C1-C4, (alcoxy en C1-C4)carbonyle, (alkyle en C1-C4)carbonyle, (alkyle en C1-C4)carbonyloxy et (alkyle en C1-C4)carbonylamino ;
R⁸ représente l'hydrogène, un groupe alkyle en C1-C4, halogénoalkyle en C1-C4, alcényle en C3-C5, halogénoalcényle en C3-C5, alcynyle en C3-C5 ou alkylsulfonyle en C1-C4 ;
R⁹ représente l'hydrogène, un groupe cyano, alkyle en C1-C4, alcoxy en C1-C4 ou alkylthio en C1-C4 ;
R¹⁰ représente l'hydrogène, un groupe alkyle en C1-C4, halogénoalkyle en C1-C4, cyanoalkyle en C1-C4, di-(alkyle en C1-C4)amino-alkyle en C1-C4, (alcoxy en C1-C4)alkyle en C1-C4, (alkylthio en C1-C4)alkyle en C1-C4, (alkyle en C1-C4)carbonyle, (alcoxy en C1-C4)carbonyle, di(alkyle en C1-C4)aminocarbonyle, (halogénoalkyle en C1-C4)carbonyle, alcényle en C3-C5, halogénoalcényle en C3-C5, di-(alkyle en C1-C4)amino-alcényle en C3-C5, (alcoxy en C1-C4)alcényle en C3-C5, (alkylthio en C1-C4)alcényle en C3-C5, alcynyle en C3-C5, halogénoalcynyle en C3-C5, cycloalkyle en C3-C7, qui peut porter éventuellement un à trois groupes alkyle en C1-C3,
ou bien un groupe phényle, benzoyle ou un groupe hétéroaryle à cinq ou six chaînons contenant un à trois hétéroatomes choisis dans le groupe consistant en trois atomes d'azote et un atome d'oxygène ou de soufre, les cycles phényle et hétéroaryle pouvant être non substitués ou pouvant porter sur chaque chaînon substituable l'un des substituants suivants : nitro, cyano, halogéno, alkyle en C1-C4, halogénoalkyle en C1-C4 ou alcoxy en C1-C4 ;
R¹¹, R¹² représentent chacun, indépendamment l'un de l'autre, l'hydrogène, un groupe alkyle en C1-C4, halogénoalkyle en C1-C4, (alcoxy en C1-C4)alkyle en C1-C4, alcényle en C3-C5, halogénoalcényle en C3-C5, alcynyle en C3-C5, halogénoalcynyle en C3-C5, cycloalkyle en C3-C7, qui peut porter éventuellement un à trois groupes alkyle en C1-C3, ou phényle qui peut porter éventuellement, sur chaque chaînon substituable, l'un des substituants suivants : nitro, cyano, halogéno, alkyle en C1-C4, halogénoalkyle en C1-C4 ou alcoxy en C1-C4,
ou bien R¹¹ et R¹² forment ensemble et avec l'atome de carbone commun auquel ils sont fixés un cycle saturé de trois à huit chaînons qui, en plus des chaînons méthylène, peut contenir éventuellement un ou deux chaînons cycliques oxygène, soufre et/ou aza, le cycle pouvant être non substitué ou pouvant porter un à quatre groupes alkyle en C1-C4 ;
R¹³ représente un groupe alkyle en C1-C4, halogénoalkyle en C1-C4, cyano-alkyle en C1-C4, di-(alkyle en C1-C4)amino-alkyle en C1-C4, (alcoxy en C1-C4)alkyle en C1-C4, (alkylthio en C1-C4)alkyle en C1-C4, alcényle en C3-C5, halogénoalcényle en C3-C5, di-(alkyle en C1-C4)amino-alcényle en C3-C5, (alcoxy en C1-C4)alcényle en C3-C5, (alkylthio en C1-C4)alcényle en C3-C5, alcynyle en C3-C5, halogénoalcynyle en C3-C5, cycloalkyle en C3-C7 qui peut porter éventuellement un à trois groupes en C1-C3, un groupe phényle ou un groupe hétéroaryle à cinq ou six chaînons contenant un à trois hétéroatomes choisis dans le groupe consistant en trois atomes d'azote et un atome d'oxygène ou de soufre, le cycle phényle et le cycle hétéroaryle pouvant être substitués ou pouvant porter sur chaque chaînon cyclique substituable l'un des substituants suivants : nitro, cyano, halogéno, alkyle en C1-C4, halogénoalkyle en C1-C4 ou alcoxy en C1-C4 ;
X et Z représentent chacun, indépendamment l'un de l'autre, une chaîne alkylène en C1-C4 qui peut être non substituée ou peut porter un à quatre substituants choisis chacun parmi les halogènes, les groupes alkyle en C1-C4, halogénoalkyle en C1-C4 et alcoxy en C1-C4 ;
ainsi que les sels des composés I convenant pour les applications agricoles.

2. Utilisation des dérivés de l'acide 5-pyrazolylbenzoïque I et de leurs sels convenant pour les applications agricoles, selon la revendication 1, en tant qu'herbicides ou pour la dessiccation/défoliation de végétaux.

3. Produit herbicide contenant une quantité herbicide efficace d'au moins un dérivé de l'acide 5-pyrazolylbenzoïque de formule I ou d'un sel de I convenant pour les applications agricoles, selon la revendication 1, et au moins un véhicule inerte liquide et/ou solide et, si on le désire, au moins une substance tensio-active.

4. Produit pour la dessiccation et/ou la défoliation de végétaux contenant une quantité dessiccante et/ou défoliante efficace d'au moins un dérivé de l'acide 5-pyrazolylbenzoïque de formule I ou d'un sel de I convenant pour les applications agricoles, selon la revendication 1, et au moins un véhicule inerte liquide et/ou solide ainsi que, si on le désire, au moins une substance tensio-active.

5. Procédé de préparation de produits à activité herbicide, caractérisé par le fait que l'on mélange une quantité herbicide efficace d'au moins un dérivé de l'acide 5-pyrazolylbenzoïque de formule I ou d'un sel de I convenant pour les applications agricoles, selon la revendication 1, avec au moins un véhicule inerte liquide et/ou solide ainsi que, si on le désire, au moins une substance tensio-active.

6. Procédé de préparation de produits à activité dessiccante et/ou défoliante, caractérisé par le fait que l'on mélange une quantité dessiccante et/ou défoliante efficace d'au moins un dérivé de l'acide 5-pyrazolylbenzoïque de formule I ou d'un sel de I convenant pour les applications agricoles, selon la revendication 1, avec au moins un véhicule inerte liquide et/ou solide ainsi que, si on le désire, au moins une substance tensio-active.

7. Procédé pour combattre les croissances de végétaux indésirables, caractérisé par le fait que l'on fait agir une quantité herbicide efficace d'au moins un dérivé de l'acide 5-pyrazolylbenzoïque de formule I ou d'un sel de I convenant pour les applications agricoles, selon la revendication 1, sur les végétaux, leur habitat ou leurs semences.

8. Procédé pour la dessiccation et/ou la défoliation de végétaux, caractérisé par le fait que l'on fait agir une quantité dessiccante et/ou défoliante efficace d'au moins un dérivé de l'acide 5-pyrazolylbenzoïque de formule I ou d'un sel de I convenant pour les applications agricoles, selon la revendication 1, sur les végétaux.
